# EUROPEAN PATENT SPECIFICATION

(11) **EP 2 604 272 B1**
(45) Date of publication and mention of the grant of the patent: **02.08.2017**
(21) Application number: 11816038.1
(22) Date of filing: 30.05.2011
(51) Int. Cl.: A61K 36/074, A61P 3/00, A61P 9/00

(54) **EFFECTIVE FRACTION FROM THE FRUITING BODIES OF GANODERMA LUCIDUM, EXTRACTION METHOD, USE AND PREPARATION THEREOF**
EFFEKTIVE FRAKTION AUS DEM FRUCHTKÖRPER VON GANODERMA LUCIDUM, EXTRAKTIONSVERFAHREN, ANWENDUNG DAVON UND PRÄPARAT DARAUS
FRACTION EFFICACE DE CORPS FRUCTIFÈRES D'UN POLYPORE LUCIDE, PROCÉDÉ D'EXTRACTION, UTILISATION ET PRÉPARATION DE CELLE-CI

(30) Priority: 09.08.2010 CN 201010248437
(43) Date of publication of application: 19.06.2013
(73) Proprietor: Fudan University, Yangpu, Shanghai 200433 (CN); Yueyang Hospital of Integrated Traditional Chinese And Western Medicine, Shanghai University of Traditional Chinese Medicine, Shanghai 200437 (CN)
(72) Inventor: ZHOU, Ping, Shanghai 200433 (CN); YANG, Hongjie, Shanghai 200437 (CN); TENG, Baosong, Shanghai 200433 (CN); WANG, Chendong, Shanghai 200433 (CN)
(74) Representative: Schrell, Andreas
(86) International application number: PCT/CN2011/074926
(87) International publication number: WO 2012/019479

(56) References cited:
- CN-A- 101 357 951
- JP-A- 2005 263 658
- US-A1- 2003 095 981
- XIAOPING C ET AL: "Free radical scavenging of Ganoderma lucidum polysaccharides and its effect on antioxidant enzymes and immunity activities in cervical carcinoma rats", CARBOHYDRATE POLYMERS, APPLIED SCIENCE PUBLISHERS, LTD. BARKING, GB, vol. 77, no. 2, 10 June 2009 (2009-06-10), pages 389-393, XP026088424, ISSN: 0144-8617, DOI: 10.1016/J.CARBPOL.2009.01.009 [retrieved on 2009-01-20]
- HUANG SHENG-QUAN ET AL: "Optimization of alkaline extraction of polysaccharides from Ganoderma lucidum and their effect on immune function in mice", MOLECULES, vol. 15, 25 May 2010 (2010-05-25), pages 3694-3708, XP002720846,
- LUO,LIXIN ET AL.: 'The Isolation and Purification of Ganoderma Lucidum Polysaccharide' SCIENCE AND TECHNOLOGY OF FOOD INDUSTRY no. 3, 1998, pages 4 - 7, XP009176446
- XIN,XIAOHEI ET AL.: 'Chemical Research on the Ganoderma Lucidum Polysaccharide' EDIBLE FUNGI OF CHINA vol. 15, no. 3, 1996, pages 14 - 15, XP008167951
- LIAO,WENBIN: 'Research Survey in Pharmacological Action of Ganoderma Lucidum Polysaccharide' CHINESE JOURNAL OF TRADITIOANAL VETERINARY SCIENCE 2009, pages 590 - 593, XP008168541

## Description

### FIELD OF THE INVENTION

The invention relates to the field of medicine or health care product, more specifically to the hypoglycemic effective fraction from Ganoderma lucidum fruiting body, extraction methods and uses thereof.

### BACKGROUND OF THE INVENTION

Ganoderma lucidum [Ganoderma lucidum (Fr.) Karst] is a kind of basidiomycetes Ganoderma fungi. It has been called "immortal grass", "lucky plant" since ancient time, and is a precious traditional Chinese medicine for strengthening and consolidating body resistance, nourishing the strength, and has a long history of Chinese medicine with extremely high medicinal value. "Shennong's Herbal Classic" in the eastern Han dynasty ranked Ganoderma lucidum as top grade of herbal which benefits "heart", "spirit", "liver"" and "muscles and bones". "Compendium of Materia Medica" indicated Ganoderma lucidum capable of "nourishing the strength", "prolonging life", "benefiting joint", "curing deafness", etc. Modern medicine has proven that Ganoderma lucidum contains many physiologically active substances, can regulate and enhance human immunity, lower blood pressure, glucose and lipid.

In the research filed of extracts of Ganoderma lucidum, scientists have isolated more than 150 kinds of extracts from Ganoderma lucidum in recent years. The extracts are divided into 10 categories: triterpenes, polysaccharides, nucleosides, furans, sterols, alkaloids, amino acids, proteins, fats, organic germanium and inorganic ions, etc. Among them, small molecular triterpenes and macromolecular polysaccharides are dominant components, and valuable for the pharmacology. Their pharmacological functions mainly involve the regulation of biological immune, prevention and treatment of tumor diseases, improvement of the body hypoxia tolerance, elimination of free radicals, etc.

Diabetes mellitus is a type of clinical syndrome caused by the interaction between genetic and environmental factors. The absolute or relative deficiency of insulin secretion as well as the decrease in sensitivity of target cells to insulin lead to a series of the metabolic disorders related to sugar, protein, fat, water and electrolyte. Hyperglycemia is a main characteristic in clinic, which can cause the damage of many systems. Serious illness and stress may occur with acute metabolic disorders such as ketoacidosis. The serious complications such as coronary heart disease, cerebrovascular disease, blindness, acral gangrene occur more significantly in diabetic patients than that in non-diabetic patients. Therefore, diabetes and its complications seriously threatening human health have become a worldwide public health problem.

Generally, diabetes are divided into two types, type I diabetes (insulin-dependent diabetes mellitus, IDDM) and type II diabetes (non-insulin-dependent diabetes mellitus, NIDDM). More than 90% of diabetes are type II. WHO predicted that the number of diabetic patients will be increased from 135 million in 1995 to 300 million in 2025 due to the aging population, obesity, unhealthy diet and lack of exercises.

Type II diabetes is also dependent on the genetic and environmental factors, and shows considerably heterogeneity. The pathogenesis is diverse and complicated. Different patients show different symptoms. In general, the symptoms are classified into the relative lack of insulin secretion and the insulin resistance. Insulin resistance refers to the decreases in sensitivity and/or responsibility to the insulin, and is dominant pathologic and physiological basis for the diabetes and metabolic syndrome. Insulin resistance, the decreased glucose tolerance, type II diabetes, obesity, dyslipidemia, nonalcoholic fatty liver, coronary heart disease and other clinical abnormalities are closely related. Insulin activates the tyrosine kinase in intracellular β subunit of receptor by binding extracellular α-subunit of receptor, leading to the regulation of autophosphorylation of the crucial tyrosine residues in the structural domain, thereby completely activating the tyrosine kinase of insulin receptor, and then the tyrosine kinase of insulin receptor transmits the signal by phosphorylating its substrate. Along with understanding more about the reversible tyrosine phosphorylation in the intracellular insulin action pathway, people have paid more attention to the role of the protein tyrosine phosphatase (PTPases) in balancing the phosphorylation level of related protein tyrosine in the pathway. PTPases may play many roles in the pathway, for instance, dephosphorylate the insulin receptor (IR) which is activated by the phosphorylation, thereby reducing the activity of the receptor kinase; or dephosphorylate the protein tyrosine residues in the insulin receptor substrate 1 (IRS-1), insulin receptor substrate 2 (IRS-2) and Shc, etc., thereby negatively regulating the insulin receptor pathway.

Protein tyrosine phosphatase-1B (PTP1B) was purified and determined being bioactive PTPase with molecular weight of about 50KD firstly. Early research showed that PTP1B could dephosphorylate the insulin receptor in vitro effectively. When PTP1B from human placental was microinjected into Africa Xenopus oocytes, it reduced the oocyte maturation induced by insulin as well as the phosphorylation level of S6 peptide. Diabetes is closely related with PTP1B. PTP1B knockout mice generated by the homologous recombination grow normally with fecundity, and have high sensitivity to insulin. The high sensitivity is correlated with the increase of phosphorylation levels of insulin receptor and its substrate in liver and skeletal muscle. Therefore, increasing and extending the insulin signaling through looking for the inhibitors selective inhibiting PTP1B in the pathway becomes more and more important for the treatment of type II diabetes.

In addition, epidemiological studies also indicate that there are two glycemic indexes including fasting blood glucose level and postprandial blood glucose level, the later is particularly important because it is an important factor for the patients with impaired glucose tolerance (IGT) to develop into type II diabetes. Moreover, the postprandial blood glucose level is related to the macrovascular as well as microvascular complications in diabetes. These complications are the major causes leading to the mortality. Therefore, strict control of the postprandial blood glucose is of great significance for the prevention and treatment of diabetes. The drugs which can significantly reduce the postprandial blood glucose level would be valuable for the application.

Most of carbohydrates in food are starch and sucrose. Starches are decomposed into oligosaccharides by salivary and pancreatic α-amylase. The oligosaccharides are decomposed into glucose by α-glucosidase in the epithelial cells in small intestine brush border, and then absorbed, leading to the increased postprandial blood glucose. Therefore, inhibition of both α-amylase and α-glucosidase activity is an effective way for lowering postprandial blood glucose.

When taken with meals, α-amylase inhibitor binds α-amylase in saliva and pancreatic juice competitively with starch, occupying the same sites in the enzyme as starch doing, and blocking the digestion for starch. The undigested carbohydrates are transported to the middle-low segment of small intestine and colon, thereby allowing the carbohydrates digested and absorbed in the whole small intestine, and delaying and prolonging the absorption of the postprandial monosaccharides (glucose), therefore, the postprandial blood glucose levels are significantly reduced. α-glucosidase inhibitors were developed in later period of 1970s' as new oral hypoglycemic drugs, and have been recommended three times as first-line drugs to reduce postprandial blood glucose by the medication guide for diabetes in Asia-Pacific region. α-glucosidase inhibitors can inhibit competitively α-glucosidase activity, defer or prevent the digestion of carbohydrates, delay the absorption of glucose from the disaccharides, oligosaccharides and polysaccharides, effectively delaying and reducing the postprandial hyperglycemia. They can reduce the fasting blood glucose levels after long-term use, and are applicable to both type I and type II diabetes mellitus.

At present, the normally used orally western medicines in clinic for treatment of diabetes mellitus are mainly sulfonylureas (D860, Glibenclamide, Gliclazide, Glipizide, Gliquidone, Glurenorm, etc.), double guanidines (phenformin, metformin, etc.), α-glucosidase inhibitors (acarbose) and insulin sensitizers (thiazolidinediones). They are less effective or have serious side effects on the liver and kidney after long time of administration, also cause the hypoglycemia reaction, and even endanger the life. Although insulin is special effective for treatment of diabetes, it would produce antibodies after long time of administration, leading to the decrease in biological activity, and the increase in dosage. The traditional Chinese medicines can overall reduce and delay the diabetic complications on the theory of differentiation of the disease syndrome. Therefore, study on the antidiabetic drugs screened from natural products is important for social and applicable values.

Ganoderma lucidum has sweet taste and neutral nature, is helpful for whole body and strengthening and consolidating body resistance when absorbed into five viscera. The spleen is related to the phlegm. Ganoderma is good for spleen and can remove the phlegm and dampness, which is the therapeutic principle of traditional Chinese medicine for the insulin resistance. However, the study of Ganoderma lucidum extracts and their hypoglycemic effect is still insufficient, and so far no effective extract from Ganoderma lucidum was reported being significantly hypoglycemic.

### SUMMARY OF THE INVENTION

The present invention is defined by the claims. In the summary of the invention, there are a series of simplified definitions which will be further described in detail in the description of embodiments. The summary of the invention does not attempt to limit the key and necessary features in the protected technical solution, moreover, not attempt to determine the protection scope in the technical solutions to be claimed.

One of the objects of the present invention is to provide a method for extraction of an effective fraction from Ganoderma lucidum fruiting body (the effective fraction was not reported before, and firstly named as FYGL by the inventors). A specific effective fraction FYGL from Ganoderma lucidum is selectively extracted through the method. Our research has proved that the effective fraction can inhibit considerably the activity of protein tyrosine phosphatase-1B, α-amylase and α-glucosidase, therefore, can be used for the treatment of diabetes and diabetes-related diseases as well as the daily health care for the hypoglycemic purpose.

The second object in the present invention is to provide an effective fraction FYGL extracted from Ganoderma lucidum fruiting body.

The third object in the present invention is to provide use of the effective fraction FYGL as the sole active component in manufacturing pharmaceuticals.

The fourth object in the present invention is to provide pharmaceutical preparations or health care products comprising the effective fraction FYGL.

Descriptions of various technical solutions according to the present invention are provided hereafter.

The first aspect of the present invention relates to the effective fraction FYGL in Ganoderma lucidum fruiting body, wherein FYGL is an extract from Ganoderma lucidum fruiting body, and has a half inhibitory concentration, i.e., IC₅₀ equal to 80 µg/mL or less on protein tyrosine phosphatase-1B activity.

The second aspect of the present invention provides use of the effective fraction FYGL in Ganoderma lucidum fruiting body according to the first aspect of the present invention as the sole active component for manufacturing a pharmaceutical, which is used for the treatment or prevention of at least one selected from diabetes and metabolic syndrome diseases; or for the treatment or prevention of at least one disease associated with diabetes or metabolic syndrome, selected from atherosclerosis, atherosclerosis, obesity, hypertension, hyperlipidemia, fatty liver disease, kidney disease, neurological disease, retinopathy, foot ulcer or cataract; or for the treatment of at least one disease selected from hyperlipidemia, obesity or cachectic disease.

The third aspect of the present invention provides use of the effective fraction FYGL in Ganoderma lucidum fruiting body according to the first aspect of the present invention as the sole active component for manufacturing a pharmaceutical, which is used for the treatment of diseases related to at least one enzyme selected from protein tyrosine phosphatase-1B, α-amylase and α-glycosidase.

The fourth aspect of the present invention provides a pharmaceutical preparation comprising the effective fraction FYGL in Ganoderma lucidum fruiting body according to the first aspect as an active component and one or more pharmaceutically acceptable carriers, and excluding any other extract from Ganoderma lucidum fruiting body.

The fifth aspect of the present invention provides a health care product comprising the effective fraction FYGL in Ganoderma lucidum fruiting body according to the first aspect as an active component and one or more edible carriers, and excluding any other extract from Ganoderma lucidum fruiting body.

The sixth aspect of the present invention provides a method for extraction of the effective fraction FYGL from Ganoderma lucidum fruiting body, wherein the effective fraction FYGL has a half inhibitory concentration, i.e., IC₅₀ equal to 80 µg/mL or less on protein tyrosine phosphatase-1B activity, wherein the method comprises that: a degreased product of Ganoderma lucidum fruiting body is extracted by alkaline extraction at 0-20°C, or the degreased product is extracted by water to obtain filtered residues and/or residues which are then extracted by alkaline extraction at 0-20°C, so as to provide an extract from alkaline extraction of Ganoderma lucidum fruiting body; the extract is dialyzed to remove small molecules; and then the dialyzed extract is directly subject to a drying treatment so as to obtain the effective fraction FYGL, or the dialyzed extract is subject to a separating treatment before the drying treatment, so as to obtain the effective fraction FYGL.

The seventh aspect in the present invention provides a method for extraction of the effective fraction FYGL from Ganoderma lucidum fruiting body, wherein the method comprises: a degreased product of Ganoderma lucidum fruiting body is extracted by alkaline extraction at 0-20°C, or the degreased product is extracted by water to obtain filtered residues and/or residues which are then extracted by alkaline extraction at 0-20°C, so as to provide an extract from alkaline extraction of Ganoderma lucidum fruiting body; the extract is dialyzed to remove small molecules; and then the dialyzed extract is directly subject to a drying treatment so as to obtain the effective fraction FYGL, or the dialyzed extract is subject to a separating treatment before the drying treatment, so as to obtain the effective fraction FYGL.

The eighth aspect of the present invention provides an effective fraction FYGL from Ganoderma lucidum fruiting body, prepared by any of the above extraction methods.

The research both in vitro and in animal test showed that the effective fraction FYGL can inhibit significantly the activity of protein tyrosine phosphatase-1B (PTP1B), α-amylase and α-glycosidase. Therefore, it can be inferred that FYGL will have an obvious hypoglycemic effect in human body, and thus can be used for the treatment or prevention of diabetes and metabolic syndrome or those diseases or symptoms related to diabetes mellitus and metabolic syndrome. In addition, the above extraction methods are simple, low cost and convenient to be applied to the industry, leading to the good economic benefits.

The inventors analyzed the effective fraction FYGL extracted from Ganoderma lucidum fruiting body using phenol-sulfuric acid method and Lowry method (Lowry). It can be inferred base on the results that the effective fraction contains polysaccharide and protein components. When used for analyzing the monosaccharide of the polysaccharide, ion chromatographic analysis indicated that the monosaccharide residues in the polysaccharide are mainly glucose, arabinose, xylose, rhamnose, galactose and fructose units. When the protein components are analyzed by amino acid analyzer, the result indicated that there are 19 natural amino acid residues in the composition of the proteins components.

### DESCRIPTION OF THE DRAWINGS

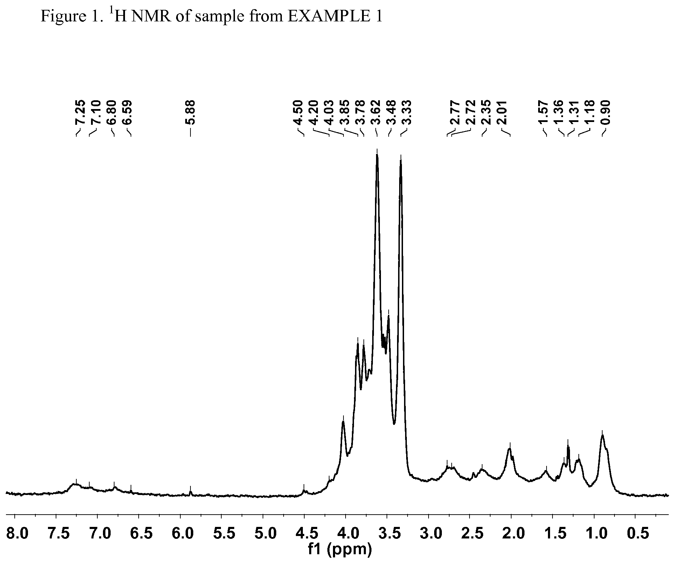

Figure 1 shows ¹H NMR spectrum of the product obtained from Example 1.

### DETAILED DESCRIPTION OF EMBODIMENTS

In the following description, the details of the embodiments are described for thoroughly understanding the invention. In other examples, some well-known technical features are not described in order to avoid confusion with the invention.

In order to understand thoroughly the invention, the detailed procedures will be described below for the extraction of the effective fraction FYGL from Ganoderma lucidum fruiting body. Obviously, the implementation of the invention is not limited to the specific details with which those skilled in the art are familiar. Some of preferable examples of the invention are described in detail below, however, in addition to these detailed descriptions, there are also other embodiments of the invention elsewhere.

### Method for extraction of the effective fraction FYGL

Firstly, according to one embodiment of the invention, a method for extraction of the effective fraction FYGL from Ganoderma lucidum fruiting body is provided herein. The method includes: a degreased product of Ganoderma lucidum fruiting body is extracted by alkaline extraction at 0-20°C, or the degreased product is extracted by water to obtain filtered residues and/or residues which are then extracted by alkaline extraction at 0-20°C, so as to provide an extract from alkaline extraction of Ganoderma lucidum fruiting body; the extract is dialyzed to remove small molecules; and then the dialyzed extract is directly subject to a drying treatment so as to obtain the effective fraction FYGL, or the dialyzed extract is subject to a separating treatment before the drying treatment, so as to obtain the effective fraction FYGL.

The above technical solution can be interpreted as the four embodiments below:
EMBODIMENT 1. The method includes the following procedures: the degreased product from Ganoderma lucidum fruiting body is extracted through alkaline extraction at 0-20°C to provide an extract from alkaline extraction of Ganoderma lucidum fruiting body; the resulting extract from the alkaline extraction is dialyzed to remove small molecules; and then the dialyzed extract is directly subject to a drying treatment, so as to obtain the effective fraction FYGL.
EMBODIMENT 2. The method includes the following procedures: the degreased product from Ganoderma lucidum fruiting body is extracted through alkaline extraction at 0-20°C to provide an extract from alkaline extraction of Ganoderma lucidum fruiting body; the resulting extract from alkaline extraction is dialyzed to remove small molecules; and then the dialyzed extract is subject to a separating treatment, and then a drying treatment, so as to obtain the effective fraction FYGL.
EMBODIMENT 3. The method includes the following procedures: the degreased product is extracted by water to obtain filtered residues and/or residues which are then extracted through alkaline extraction at 0-20°C to provide an extract from alkaline extraction of Ganoderma lucidum fruiting body; the resulting extract from the alkaline extraction is dialyzed to remove small molecules; and then the dialyzed extract is subject to a separating treatment, and then a drying treatment, so as to obtain the effective fraction FYGL.
EMBODIMENT 4. The method includes the following procedures: the degreased product is extracted by water to obtain filtered residues and/or residues which are then extracted through alkaline extraction at 0-20°C to provide an extract from alkaline extraction of Ganoderma lucidum fruiting body; the resulting extract from the alkaline extraction is dialyzed to remove small molecules; and then the dialyzed extract is directly subject to a drying treatment, so as to obtain the effective fraction FYGL.

In one of preferable embodiments in this invention, the effective fraction FYGL consists essentially of materials with weight-average molecular weights from 1kDa to 100kDa. In the preferable extract, the concentration of active component in the effective fraction is further increased.

In one of preferable embodiments in this invention, after the separation treatment, the drying treatment includes at least one selected from spray-drying, vacuum-drying, freeze-drying or atmospheric drying. Although different drying methods do not influence yield of the extract, the mild method, such as vacuum and/or atmospheric drying, would be preferable for prevention of the extracts from the damage; but the spray-drying and freeze-drying methods would be preferred for improving production efficiency.

In one of preferable embodiments in this invention, the degreased products are obtained by degreasing the Ganoderma lucidum fruiting body using alcohols or aqueous solution of alcohols. The degreasing preferably involves a drying treatment, so as to increase the efficiency in the following procedures.

For the raw materials used, the experimental results of the inventors show that the effective fraction FYGL is present in the Ganoderma lucidum fruiting bodies from various places, and the extraction method of the invention is highly selective to the effective fraction FYGL. Therefore, Ganoderma lucidum fruiting body used in the extraction method in the invention is not limited to certain regions of Ganoderma lucidum. In other words, the Ganoderma lucidum fruiting body can be collected from all places of the country, either from genuine medicinal materials or from common medicinal materials; either from wild ones or artificial cultivated ones. For example, one can collect Ganoderma lucidum fruiting body from provinces of Jilin, Zhejiang and others, also can buy from dealers (such as Shanghai Leiyunshang). In order to improve the efficiency of degreasing, the shredders and other equipments can be used to crush the Ganoderma lucidum fruiting body before degreasing.

The degreasing agents used for the degreasing can be alcohol or alcohol aqueous solution. Ethanol or ethanol aqueous solution would be preferred for easy availability. In terms of the production efficiency, the ethanol concentration of its aqueous solution is preferably 50 v/v% or more, more preferably 70 v/v% or more, most preferably 95 v/v% or more. Number of degreasing times can be 1-5. This number can be changed properly based on the alcohol concentration of the alcohol solution, as long as the lipid compounds in the fruiting body can be substantially removed. For example, 95 v/v% ethanol aqueous solution can be used for degreasing Ganoderma lucidum fruiting body 1-3 times. In terms of degreasing temperature, the temperature can be room temperature, but preferably slightly higher than room temperature such as 40°C or more. For instance, 95 v/v% ethanol aqueous solution can be used for degreasing at boiling temperature (about 78°C) under reflux. The degreasing time can be properly determined, for example, from 20 minutes to 10 hours, based on, such as, raw material quantity, degreasing temperature, degreasing agent concentration as well as the ratio of raw material to degreasing agent. It is noticed that the purpose of degreasing is solely to remove the lipid compounds in Ganoderma lucidum fruiting body. The general degreasing methods are well-known to a person of ordinary skill in the art; therefore, in addition to methods mentioned above, a person of ordinary skill in the art can also use those conventional experimental means to achieve this goal.

The drying step applicable to the degreased materials (residues) can be at least one selected from spray-drying, vacuum-drying, freeze-drying or drying under normal pressure. If high volatile and high concentration of alcohol solution is used as a degreasing agent, the air drying is preferred for further reducing the cost. Thus, the degreased product is obtained. In summary, the degreasing process can either include drying the degreased product or be carried out without any drying treatment so as to be directly used for the subsequent steps.

Then the degreased products are treated through alkaline extraction. The temperature for the alkaline extraction (i.e., alkaline extraction temperature) can be 0°C (ice-water bath) - 20°C. The inventors unexpectedly discovered that the lower temperature of alkaline extraction selected in the mentioned range can significantly increase the activity of the effective fraction; therefore, from this point of view, the lower the temperature of the alkaline extraction is, the higher the activity of the extract is. If the temperature of the alkaline extraction is higher than 20°C during ordinary extraction time (such as 1 hour), the effective fraction will be destroyed, possibly leading to the covalent bonds broken between protein and polysaccharide, and then to the formation of small molecular compounds, which will be removed in the subsequent dialysis step, resulting in the decrease of the yield of the final extract, the reduction of the activity, and the loss of effective fraction; namely, the half inhibitory concentration of IC₅₀ of the extract on the activity of protein tyrosine phosphatase-1B is larger than 80µg/mL. On the other hand, the lower the temperature is, the slower the speed of alkaline extraction is, leading to more times of alkaline extraction required; moreover, too much energy is spent on the alkaline extraction, resulting in diseconomy. Therefore, more preferred temperature of alkaline extraction is 4-15°C. For example, in one preferred embodiment, it takes 10 hours or more, and more preferably 12-24 hours for the alkaline extraction with the number of 1-5 times at 10°C. However, it would take more than 24 hours for the alkaline extraction at 0°C with the same extraction efficiency as that at 10°C.

The alkali solution may be an aqueous solution comprising at least one substance selected from sodium carbonate, sodium bicarbonate, potassium hydroxide, sodium hydroxide or ammonia. Preferably, the solution may be the sodium carbonate or sodium bicarbonate solution with concentration of 0.5M or more; or potassium hydroxide, sodium hydroxide or ammonia solution with concentration of 0.1-2M. In order to obtain a higher yield, the extraction time can be appropriately extended (for example, extended to 15 hours or more), and/or the alkaline concentration may be increased (for example, the concentration of sodium hydroxide may be increased to 1.5M or more, and the concentration of sodium carbonate may be increased to 2M or more). In terms of the enhancement of production efficiency, the concentration of 1.0M or more of sodium carbonate, or 0.5-2M of sodium hydroxide or potassium hydroxide or ammonia is more preferred. The higher the concentration is, the higher the efficiency is. But over-high concentration may also lead to the targeted extracts impaired and the subsequent procedures complicated, and thus is not preferred. Then, the extracted solutions from the alkaline extraction may be filtered. Preferably, the filtrate may be neutralized by acid agents such as hydrochloric acid or acetic acid, and then concentrated for the following steps. Of course, a person of ordinary skill in the art would understand that those procedures as filtering, neutralizing and concentrating can also be omitted according to the actual situation.

Although the target extract of the invention exists in the extracts of alkaline extraction, the water extraction of the degreased products can also be carried out firstly, and then the remaining filtered residues and/or residues after the water extraction may be treated through alkaline extraction to obtain an extract from alkaline extraction. Of course, part of the degreased products can also be treated by water extraction, and then the resulting residues and the filtered residues together with other degreased products can be treated through the alkaline extraction to obtain an extract. In short, no matter what means and procedure sequence is used, the only necessary thing is to obtain the extracts from the alkaline extraction of the degreased products. Therefore, a person of ordinary skill in the art can choose the proper procedures according to the actual situation. For example, in a preferred embodiment, the degreased products are treated through water extraction to obtain the extracts which may contain active fractions applicable to other purposes, and then the resulting filtered residues and the residues after water extraction can be treated through the alkaline extraction to obtain an extracts used to produce the effective fraction FYGL according to the invention. In other words, the raw materials used for alkaline extraction can be the filtered residues and/or residues obtained by water extraction. Since those raw materials are commonly discarded as wastes during the water extraction, the above embodiment can significantly reduce the cost of the alkaline extraction.

During water extraction, deionized water, for example, can be used for the extraction. The extraction temperature can be the temperature of conventional water extraction for traditional Chinese medicine, e.g., 50-80°C, but in order to improve the production efficiency, the reflux with boiling water is preferred for the extraction. The number of extraction times can be 1-5, and extraction time is 1-5 hours, depending on the circumstances.

After the extract from Ganoderma lucidum fruiting body is obtained by alkaline extraction as described above, a dialysis of the extract is required to remove small molecules, in order to improve the concentration of the resulting effective ingredient. The small molecules include but are not limited to: small inorganic molecules, small water-soluble organic molecules, small molecules of proteins or sugars, etc. Among them, small inorganic molecules may include the alkali doped in the extract from alkaline extraction, the formed salt after neutralization of the base and the possible hydrochloric acid. For such intermediate extract mixtures from Ganoderma lucidum fruiting body, those skilled in the art would know the meaning of small molecules to be removed. What is clear herein is that the effective fraction FYGL according to the invention is substantially free of such kinds of small molecules that are not active components and potentially interfere with the subsequent separation, thereby reducing the concentrations of active components in the final product. Therefore, those small molecules are preferably removed prior to the further separation. Dialysis is a commonly-used means to remove small molecules in this field. Its advantage is low-cost in the removal of small molecules existing in the biological macromolecules without loss of active components. Preferably in the extracting method of the invention, the extracts or the concentrated ones from the alkaline extraction are put into a dialysis tube with molecular weight cut-off of 1kDa-3kDa (i.e., any dialysis tubes with molecular weight cut-off between 1kDa-3kDa, such as 1kDa and 3kDa), and then the tube is placed in the deionized water for 1-3 days of dialysis. In addition to dialysis, however, those skilled in the art can also use other suitable means (for example, isolation with column chromatography, ultrafiltration membrane with molecular weight cut-off 1kDa-3kDa and so on) to remove small molecules in the extracts of alkaline extraction, as long as the small molecules can be removed. If molecular weight cut-off value of the dialysis tube or ultrafiltration membrane is greater than 3kDa, the effective fraction may be lost, but the molecular weight cut-off less than 1kDa would allow the inactive small molecules, such as alkaline-hydrolyzed proteins and polysaccharides, retained in the extracts, significantly reducing the concentration of active component, and influencing the determination of protein and polysaccharide contents in the effective component.

Afterwards, the crude products without small molecules may be directly subject to the drying treatment to produce the effective fraction FYGL, or prior to the drying treatment, a separating treatment is firstly carried out in order to further extract the active components, and enhance the content of the active components in the final product. The drying treatment is at least one selected from spray-drying, vacuum-drying, freeze-drying or atmospheric drying.

In one of the preferable embodiments in this invention, the separating treatment includes:
(1) carrying out an alcohol precipitation of the dialyzed extract so as to obtain a supernatant; or
(2) fractionating the dialyzed extract in the manner of ultrafiltration by using ultrafiltration membrane with its molecular weight cut-off of 100 kDa or less, so as to remove those with molecular weight higher than the cut-off value. That is, the specific extracts with a given molecular weight range are collected mainly through alcohol precipitation or ultrafiltration. In the present invention, the molecular weight range can be 1kDa to 100kDa, more preferably 1kDa to 50kDa, and even preferably 1kDa to 30kDa.

The inventors have found that when the method of alcohol precipitation was used, the large molecular weight components would be precipitated and removed; thereby the active components with moderate molecular weight would be obtained. The active components with moderate molecular weight can also be collected by ultrafiltration membrane with proper selection of molecular weight cut-off value. Experiments showed that the large molecular weight components with molecular weight higher than 100kDa have a significantly weak capability of inhibiting PTP1B activity, and therefore the ultrafiltration membranes with molecular weight cut-off 100kDa or less are preferably used for the ultrafiltration, and the molecular weight cut-off is more preferably 70kDa or less, further preferably 50kDa or less, especially preferably 30kDa or less, for further enhancing the concentrations of active components in the filtrate.

Ultrafiltration is a technique for the concentration and separation of macromolecules or colloids simultaneously, wherein the pressure difference as a driving force allows the liquid passing through the membrane, leading to the macromolecules or colloids blocked, while the small molecules passing through. Ultrafiltration membranes may be flat film, roll film, tubular membrane and hollow fiber membrane, etc. Generally, the porous sizes of ultrafiltration membranes are roughly within 50-10000Å which can retain the particles with radius range of 1-20nm, equivalent to a molecular weight of 300 to 300000 of proteins and polymers. The molecular weight cut-off value refers to the minimum molecular weight of solutes blocked by the membrane. The ultrafiltration membrane materials used in the invention can be cellulose and its derivatives, polycarbonate, polyvinyl chloride, polyvinylidene fluoride, polysulfone, polyacrylonitrile, polyamide, polysulfone amide, sulfonated polysulfone, cross-linked polyvinyl alcohol, modified acrylic polymer, etc. Since the film materials do not affect the composition of the blocked final product, they are not limited in this inventive method, as long as the molecular weight cut-off values are within the above-mentioned range.

Fractionation through alcohol precipitation is a conventional technique for those skilled in the art. It is a fractionation separating effective fractions away from the other components based on their different solubilities in alcohol as a polar solvent and water. The inventors have found that the fractionation through alcohol precipitation could separate the effective fraction FYGL away from the inactive components (called impurity) in the above crude extract. In the invention, the ethanol aqueous solution is preferably used to precipitate the impurities in the above crude extracts, and then the supernatant is collected. In a preferred embodiment, after dialysis duration of 2-4 days, the solution in the dialysis tube is concentrated to 1/3-1/5 of the original volume, and then 95 v/v% ethanol in volume of 2-5 times of the concentrated solution is added and the resulting solution is placed for 12 to 24 hours at room temperature. The supernatant is collected after centrifugation. Those skilled in the art can properly adjust the above conditions to obtain better extraction efficiency according to the actual situation.

For the ultrafiltration, those skilled in the art can freely select within the molecular weight cut-off range as mentioned above, a proper cut-off value, such as, 40kDa, 30kDa, 20kDa and 10kDa, etc. according to the availability of the membranes, but the preferable cut-off value is not less than 20kDa. In one of examples in the invention, the effective fraction FYGL with molecular weight of 50kDa or less was achieved by using the ultrafiltration membrane with molecular weight cut-off 50kDa. Given that the dialysis tube with molecular weight cut-off 1kDa is used previously, the extracted effective fraction FYGL herein is composed mainly of 1-50kDa components.

In order to improve the content of active component by ultrafiltration, besides the ultrafiltration membrane with molecular weight cut-off of 100kDa or less, the membranes with small molecular weight cut-off values can also be used to filter the extracts of the alkaline extraction to remove the small molecules with molecular weight less than the smaller cut-off value. For example, the extracts of the alkaline extraction may be filtered by ultrafiltration membrane with molecular weight cut-off of 10kDa or less (1kDa, 2kDa, 3kDa ...... 10kDa) to remove the components with molecular weight less than the cut-off value, and then the resulting products may be filtered by ultrafiltration membrane with molecular weight cut-off of 100kDa or less; or the extracts of the alkaline extraction may be filtered by ultrafiltration membrane with molecular weight cut-off of 100kDa or less to remove the components with molecular weight higher than the cut-off value, and then the resulting filtrate may be filtered by ultrafiltration membrane with cut-off of 10kDa or less (1kDa, 2kDa, 3kDa ... ... 10kDa). In short, in order to obtain the active fraction with any region of molecular weight distributed within 1-100kDa, those skilled in the art can use two ultrafiltration membranes with any two different cut-off values (but both within the above range) for the ultrafiltration. Also, for the separation, the ultrafiltration membranes are not necessarily required to obtain the active fraction with the mentioned molecular weight distribution. Those skilled in the art can use any other means (such as alcohol precipitation, column chromatography and so on) to achieve the same goal. Preferably, the low-limit of molecular weight cut-off value is not more than 10kDa, more preferably not more than 5kDa.

In addition, in order to prevent some impurity particles in the crude extracts blocking the ultrafiltration membrane, the crude extract is preferably filtered in advance by a filter with pore size of 2 to 15 micron (such as 3 micron, 5 micron, 7 micron, 10 micron, etc.) to further remove the larger insoluble impurities, and then the clear pre-filtrate is filtered by the ultrafiltration. Compared with the alcohol precipitation, the ultrafiltration needs relatively short time, and therefore, is preferable for the production efficiency. Moreover, the ultrafiltration membrane can be repeatedly used, more environmentally friendly than ethanol. Compared with the alcohol precipitation, the ultrafiltration can also further enhance the content of the active component in the final product, and therefore is a more preferable method.

The supernatant obtained by the molecule weight fractionation may be subject to a drying treatment so as to obtain the effective fraction FYGL according to the invention. The drying treatment includes at least one selected from spray-drying, vacuum-drying, freeze-drying or atmospheric drying.

In spite of the above, in terms of further enhancement of the activity of the effective fraction, the column chromatography is preferably carried out to select the components with lower half inhibitory concentration on the protein tyrosine phosphatase-1B activity prior to the drying treatment. The column chromatography is performed by at least one selected from the columns such as DEAE-cellulose, DEAE-Sephadex, DEAE-Sepharose or macroporous adsorption resin. In one of preferable embodiments, the eluent used is 0.1-2M NaCl aqueous solution and then 0.1-2M NaOH aqueous solution. The ratio of diameter to height of the resin column is 15-30, and its flow rate is 0.5-2.5 column volume per hour. Those skilled in the art can adjust properly those conditions according to the above teaching combined with the actual situation.

After the eluting step, the refinement may be further carried out; that is, the eluted components may be assayed by phenol-sulfuric acid method or Lowry assay, and the components with positive reaction may be collected, and then dialyzed by the dialysis tube with molecular weight cut-off 1kDa to remove the small molecules of inorganic salts and water soluble organics. Afterward, the resulting contents in the dialysis tube may be vacuum-concentrated, and dried, thereby resulting in the effective fraction FYGL in Ganoderma lucidum fruiting body.

In one of the preferable embodiments, the effective fraction FYGL is prepared by the following method:
(1) Degreasing step: a medicinal material of Ganoderma lucidum fruiting body is crushed, and then degreased with 50-100% (v/v) ethanol 1-5 times at 40-78°C for 1-5 hours every time. The degreased filtered residues are air-dried, so as to obtain a degreased product.
(2) Alkaline extraction step: the degreased product is refluxed in boiling water 1-5 times for 1-5 hours every time, and then filtered to obtain filtered residues. The filtered residues are treated through the alkaline extraction 1-5 times using 0.5M or more sodium carbonate solution or 0.1-2M sodium hydroxide solution or 0.5M-2M ammonia solution at 0-20°C (more preferably 4-15°C) for 12-24 hours each time, so as to obtain an extracted solution. The extracted solution is filtered, and then neutralized with 0.1-1M hydrochloric acid, and vacuum-concentrated, so as to obtain a concentrated solution of alkaline extraction.
   Alternatively, the degreased product is treated directly through the alkaline extraction with 1.0M or more of sodium carbonate solution or 0.5-2M sodium hydroxide solution or 1.0M or more of ammonia solution 1-5 times at 0-20°C (more preferably 4-15°C) for 12-24 hours. All the extraction solutions are combined and filtered, and then neutralized with 0.1-1M hydrochloric acid, and vacuum-concentrated, so as to obtain a concentrated solution of alkaline extraction having a smaller volume.
(3) Dialysis step: the solution of alkaline extraction is dialyzed with a dialysis tube of molecular weight cut-off 1kDa-3kDa for 1-4 days, and then the solution in the dialysis tube is concentrated to 1/3-1/5 of the original volume.
(4) Drying step: the solution of alkaline extraction obtained by the dialysis step is freeze-dried directly.
(5) Optional separating step: 95% ethanol having a volume of 2-5 times of the solution of the alkaline extraction obtained by the dialysis step is added into the solution, and the resulting solution is placed for 12-24 hours at room temperature. The supernatant is collected by centrifugalization, and dried, so as to obtain the effective fraction FYGL of Ganoderma lucidum fruiting body.
   Alternatively, the solution of alkaline extraction obtained by the dialysis step is fractioned by an ultrafiltration membrane with molecular weight cut-off 100kDa to obtain the components with molecular weight 1-100kDa, and then dried, so as to obtain the effective fraction FYGL in Ganoderma lucidum fruiting body. Optionally, the solution of alkaline extraction obtained by the dialysis step is filtered in advance by a filter with pore size of 2-15 micron to further remove the larger insoluble impurities, and then the clear pre-filtrate is filtered by the above ultrafiltration method.
(6) Optional column chromatography step: the above-mentioned effective fraction FYGL is dissolved in deionized water, and then absorbed by macroporous adsorption resin column with ratio of diameter to height of 15-30. 0.1-2M NaCl and 0.1-2M NaOH are used in succession as eluents for gradient elution. The flow rate is 0.5-2.5 column volume per hour. Further optionally, the eluted components are assayed by phenol-sulfuric acid method, and the components with positive reaction are collected and then dialyzed by a dialysis tube with molecular weight cut-off 1kDa to remove small molecules of inorganic salts and water soluble organics. Afterward, the solution in the dialysis tube is vacuum-concentrated, and dried, thereby resulting in the effective fraction FYGL of Ganoderma lucidum fruiting body.

### EFFECTIVE FRACTION FYGL

In this invention, the effective fraction FYGL refers to an extract from Ganoderma lucidum fruiting body, which has a significant activity inhibiting the protein tyrosine phosphatase-1B (PTP1B) activity. Specifically, the effective fraction has a half inhibitory concentration of IC₅₀ equal to or lower than 80µg/mL on PTP1B activity. Before this invention is proposed, such an extract from Ganoderma lucidum fruiting body as having a half inhibitory concentration of IC₅₀ equal to or lower than 80 µg/mL on protein tyrosine phosphatase-1B activity has never been reported in the prior art. The inhibitory concentration of IC₅₀ of the final product may vary with different process conditions (such as temperature, heating time, type and concentration of alkaline solution for the extraction, molecular weight cut-off value of dialysis tube, dialysis duration, ultrafiltration membranes, chromatography columns) used in the extraction methods (the inhibitory concentration of IC₅₀ values from various conditions will be described in detail below), but the inventors found, as long as the above-mentioned methods according to the invention are used, an extract from Ganoderma lucidum fruiting body, namely the effective fraction FYGL, with a half inhibitory concentration of IC₅₀ equal to or lower than 80 µg/mL on protein tyrosine phosphatase-1B activity can be obtained.

Obviously, taught by the present invention, those skilled in the art can carry out the various modifications or alternatives to get an extract from Ganoderma lucidum fruiting body with a half inhibitory concentration of IC₅₀ equal to or lower than 80 µg/mL on protein tyrosine phosphatase-1B activity. Therefore, one of embodiments in the invention provides an effective fraction FYGL from Ganoderma lucidum fruiting body with a half inhibitory concentration of IC₅₀ equal to or lower than 80 µg/mL on protein tyrosine phosphatase-1B activity. The half inhibitory concentration of IC₅₀ is preferably equal to or lower than 70 µg/mL, more preferably equal to or lower than 50µg/mL, further preferably equal to or lower than 40µg/mL, further more preferably equal to or lower than 30µg/mL, particularly preferably equal to or lower than 20µg/mL. The low limit of the half inhibitory concentration of IC₅₀ is greater than 10µg/mL, preferably higher than 0.

For the inhibitory concentration, the smaller the value is, the higher the activity of the extract is, therefore more preferred. In one of preferable embodiments in this invention, the preferable effective fraction FYGL can be those obtained by column chromatography of the molecular-weight-fractioned products. Our experiment demonstrated that the effective fraction FYGL could have a half inhibitory concentration of IC₅₀ as low as less than 20µg/mL.

Nuclear magnetic resonance (NMR) technology is used to reveal the molecular structure and motion on the different nuclear magnetic property in the substances. The nuclei in the particular external magnetic field absorb the electromagnetic wave energy. The absorption phenomenon is recorded, resulting in a nuclear magnetic resonance spectrum. As the absorption is closely related to the nuclei environment, such as ¹H nucleus environment, so the absorption features can reflect the information of molecular structure. Nuclear environment involves the functional groups, links and stereo structure in the compound. The absorption features are described by peak chemical shift (chemical shift, unit: ppm, a value referred to a reference sample), peak relative integral area, peak splitting numbers. NMR is a fingerprint of a compound with specific composition and structure, one-to-one corresponding to the molecular structure. In 1952, Swiss scientist Felix Bloch and American scientist Edwar Wills Purcell won the Nobel Prize in physics for the discovery of precise measuring method of magnetic measurement. In 1953, the first set of 30 MHz NMR spectrometer was set up, and from then the organic structures were characterized by nuclear magnetic resonance method. In 1991 and 2002, two Swiss scientists Richard R. Ernst and Kurt Wuthrich won Nobel Prize in chemistry, respectively, for the development of multidimensional NMR theory and the characteristic of biomacromolecule structures. As NMR spectrum is a fingerprint of molecular structure, the peak chemical shift values and their relative areas are not changed with the magnetic field strength, but high strength of magnetic spectrometer can improve the signal sensitivity and resolution, increase the signal intensity. The optimized experimental parameters can also allow the signal intensity increased, the noise baseline smoothed, but have no influence on the chemical shifts and peak relative areas. For liquid NMR measurement, sample is required to be dissolved in a deuterated solvent. The different deuterated solvent will affect the chemical shift. Therefore, the deuterated solvent and reference sample should be the same in the different NMR measurement for the comparison. The reference sample, such as tetramethylsilane (TMS), is commonly used in NMR measurement. TMS has only ¹H nuclear resonance peak defined as 0 ppm of chemical shift. In addition to single crystal X-ray diffraction technology, NMR technology is the most powerful tool to study the molecular structure. But the former requires single crystal which is prepared difficultly, the latter is for solution sample. Therefore NMR technology is more commonly used to solve organic molecular structure. Although nuclear magnetic resonance spectroscopy is often used as a fingerprint for the single compound, the mixture of several single compounds together in certain proportion also shows a NMR fingerprint. When effective components confirmed by biological activity experiments are obtained by extraction methods of traditional Chinese medicine, NMR fingerprint of the effective components can be used for screening extracts obtained from various extraction ways. Compared with the biological activity experiments, this screening can more quickly determine whether effective components are contained in extracts.

Based on the above theory, ¹H NMR is used in this invention to determine the structure and composition of the effective fraction FYGL. It is found in ¹H NMR spectrum that the peak integral area ratio of chemical shift range within δ=3.6-3.4 ppm to δ=3.4-3.2 ppm is 0.5-1.5; the peak integral area ratio of chemical shift range within δ=3.0-1.0 ppm to δ=3.4-3.2 ppm is 0.5-4.0. In the determination of ¹H NMR, deuterated water is used as solvent; ¹H chemical shift of tetramethylsilane is defined as 0.0 ppm as an external standard. For a better half inhibitory concentration of IC₅₀ on PTP1B activity, the peak integral area ratio of chemical shift range within δ=3.6-3.4 ppm to δ=3.4-3.2ppm is more preferably 1.2-1.5, and that of chemical shift range within δ=3.0-1.0 ppm to δ=3.4-3.2 ppm is more preferably 2-4. Most preferably, the peak integral ratio of chemical shift range within δ=3.6-3.4 ppm to δ=3.4-3.2ppm is 1.0-1.5, and that of chemical shift range within δ=3.0-1.0 ppm to δ=3.4-3.2 ppm is 2.5-4.

Basing on the relationship of polysaccharide and protein structures with NMR chemical shifts as well as on the composition analysis for monosaccharide and amino acid residues in the effective fraction FYGL of Ganoderma lucidum fruiting body, the inventors suppose that the peaks at chemical shifts within d 3.6-3.4 ppm correspond mainly to the polysaccharides which consist of monosaccharide units as glucose, xylose, rhamnose, etc.; the peaks at chemical shifts within d 3.4-3.2 ppm correspond mainly to the polysaccharides which consist of monosaccharide units as glucose and xylose; the peaks at chemical shifts within d 3.0-1.0 ppm correspond mainly to the protein or polypeptide. In addition, the peaks at chemical shifts within d 3.6-6.0 ppm are attributed to arabinose, xylose, rhamnose, galactose and fructose as well as proteins in addition to the glucose. The inventors found that in ¹H NMR spectra of the effective fraction FYGL extracted by various ways mentioned above, the peak integral area ratios of chemical shifts within given ranges are all in the above specific range, and related to the values of half inhibitory concentration of IC₅₀ (the correlations will be provided in the examples). In general, the peak integral area ratios in ¹H NMR spectrum correspond to the ratios of contents of corresponding functional groups and components, namely, the ratios are directly proportional to the contents of corresponding components. In other words, the higher the ratio, the higher the content of the former component is. Therefore, the above results show that the effective fraction FYGL contains the specific peptides or proteins and polysaccharides in certain proportions.

Moreover, based on the above results, those skilled in the art can anticipate that for any extracts of Ganoderma lucidum fruiting body, if ¹H NMR spectrum of the extract has the feature peaks with ratios of integral areas in the above ranges, the extract will have ability to significantly inhibit the activity of PTP1B. In addition, as mentioned above, the effective fraction FYGL extracted from Ganoderma lucidum fruiting body by the extraction methods according to the invention has a half inhibitory concentration of IC₅₀ equal to or lower than 80µg/mL on PTP1B activity. However, the inventors also found that although the values of IC₅₀ will be dependent on the different pathways of the extraction (Examples will provide the corresponding data), the features of ¹H NMR spectrum of all the Examples meet the above standards; therefore, the components do not significantly vary as the pathways (such as alcohol precipitation or ultrafiltration) are changed. In other words, all the extracts from Ganoderma lucidum fruiting body obtained through the above-mentioned various pathways have a similar PTP1B inhibition ability as well as a basically consistent composition.

In addition, the inventors also found that besides the above-mentioned peaks, for the preferable effective fraction FYGL, there are some typical peaks such as around (i.e., about ± 0.1 ppm. The error is resulted from the reference sample of tetramethylsilane as ether external standard or internal standard) 0.9, 1.2, 1.4, 1.6, 2.0, 2.3, 2.7, 3.4, 3.5, 3.6, 3.8, 3.9, 4.0, 4.2, 4.3, 4.5, 4.7, 4.8, 5.0, 5.3, 5.9, 6.0 and 6.6-7.6 ppm in ¹H NMR spectrum. According to the analysis of amino acid composition, there are total 19 kinds of natural amino acids (as the sample was treated by acid for the amino acid composition analysis, the tryptophan was decomposed, and could not be measured directly), the top 5 amino acids (their contents are greater than 9% of the total amino acid content) are alanine, glycine, aspartic acid, glutamate and serine. They are thought as the dominant amino acid residues in the protein moiety of the extract. The amino acid whose content is greater than 2% of the total amino acid content is threonine, phenylalanine, leucine, valine, tyrosine, isoleucine, proline, lysine, arginine, histidine, etc. The monosaccharide analysis by ion chromatography showed that there are mainly six kinds of monosaccharides which are glucose, arabinose, xylose, rhamnose, galactose, and fructose. Among them, the dominant monosaccharide is glucose, the content of which is greater than 70% of total sugar content. According to the analysis of both amino acids and monosaccharides as well as ¹H NMR along with the literature reports, ¹H NMR spectrum of FYGL was assigned in Table 1.

**Table 1 ¹H NMR assignment of FYGL**

| Chemical shift δ (ppm) | Signal assignment |
|---|---|
| 0.9 | isoleucine H_{δ} |
| 1.2 | alanine H_{β}, isoleucine H_{γ}, threonine H_{γ}, lysine H_{γ}, rhamnosus-6 deoxy-CH₃ |
| 1.4 | leucine H_{γ}, lysine H_{γ} |
| 1.6 | arginine H_{γ}, isoleucine H_{β}, leucine H_{β}, lysine H_{δ}, proline H_{γ} |
| 2.0 | glutamic acid H_{β}, glutamine H_{β}, methionine H_{δ} |
| 2.3 | methionine H_{β}, isoleucine H_{β}, glutamic acid H_{γ}, glutamine H_{γ} |
| 2.7 | lysine H_{δ}, aspartic acid H_{β}, asparagine H_{β}, tryptophan H_{β}, lysine H_{ε} |
| 3.4 | β-glucopyranose H₂, β-xylofuranose H₃ |
| 3.5 | β-glucopyranose H₄, β-xylofuranose H₂, β-rhamnopyranose H₄ or arginine H_{α}, glutamic acid H_{α}, glutamine H_{α}, methionine H_{α}, serine H_{α}, threonine H_{α}, valine H_{α}, isoleucine H_{α}, leucine H_{α} |
| 3.6 | β-glucopyranose H₃, β-arabinofuranose H_{5b}, β-xylofuranose H₄, β-rhamnopyranose H₃, β-galactopyranose H₂, β-fructofuranose H₁, β-fructopyranose H₁ or alanine H_{α}, asparagine H_{α}, glycine H_{α}, proline H_{α}, threonine H_{α} |
| 3.8 | β-glucopyranose H₅, β-xylofuranose H₅ₐ, β-galactopyranose H₃, α-fructofuranose H₁, β-fructopyranose H₆ or asparagine H_{α}, cysteine H_{α}, lysine H_{α} |
| 3.9 | β-glucopyranose H₆ₐ, β-rhamnopyranose H₂, β-galactopyranose H₅, H₆, α-fructopyranose H₆, β-fructofuranose H₅, H₆, β-fructopyranose H₃ or histidine H_{α}, phenylalanine H_{α}, tryptophan H_{α}, tyrosine H_{α} |
| 4.0 | β-arabinofuranose H₃, β-xylofuranose H₅ₐ, β-galactopyranose H₄, β-fructopyranose H₄ or threonine H_{β} |
| 4.2 | β-arabinofuranose H₄, β-xylofuranose H_{5b}, α-fructofuranose H₄, H₅, β-fructopyranose H₅ |
| 4.3 | β-arabinofuranose H₅ₐ, α-fructofuranose H₃, β-fructofuranose H₃, H₄ |
| 4.5 | β-pyran dextran H_{6b} |
| 4.7 | β-arabinofuranose H₂ |
| 4.8 | β-glucopyranose H₁, β-xylofuranose H₁, β-galactopyranose H₁ |
| 5.0 | β-arabinofuranose H₁ |
| 5.3 | α-rhamnopyranose H₁, α-galactopyranose H₁ |
| 5.9 | α- arabinofuranose H₁ |
| 6.0 | α-glucopyranose H₁ |
| 6.6-7.6 | Protons on aromatic rings of histidine, phenylalanine, tyrosine and tryptophan residues |

### APPLICATIONS OF THE EFFECTIVE FRACTION FYGL

As mentioned above, the effective fraction FYGL is an extract from Ganoderma lucidum fruiting body, which can inhibit considerably the activity of protein tyrosine phosphatase-1B (PTP1B). The inhibitory concentration of IC₅₀ is equal to or lower than 80µg/mL on protein tyrosine phosphatase-1B activity. In addition, the effective fraction has inhibitory ability on α-amylase and α-glucosidase activities in some extent, and therefore, can be used in the preparation of pharmaceuticals for treatment of diseases related to those enzymes. Specifically, the effective fraction FYGL can be applied into the treatment or prevention of at least one selected from diabetes and the metabolic syndrome diseases. Those diseases can be at least one selected from diabetes or metabolic syndrome related diseases as atherosclerosis, atherosclerosis, obesity, hypertension, hyperlipidemia, fatty liver disease, kidney disease, neurological disease, retinopathy, foot ulcer or cataract. The pharmaceutical preparation of this invention can contain any of the above-mentioned effective fraction FYGLs as its active component and one or more pharmaceutically acceptable carriers. The pharmaceutical preparation can be tablet, capsule, granule, pill, injection, oral liquid, suspension agent, guttate pills, micro pill, spray, aerosol, Babu plaster or paster. The effective fraction FYGL is used either as the sole active component or as a mixture with one or more other effective components in the pharmaceutical preparation. Whether the effective fraction FYGL is used alone or in combination, its dosage can be properly adjusted according to the actual situation. Its pharmaceutical preparation can be prepared according to the conventional methods in this field. Since preparation methods of a pharmaceutical containing effective components of traditional Chinese medicine are well-known conventional technologies for those skilled in the art, the methods will not be described in detail herein.

In addition to the usage for the pharmaceutical preparation, the effective fraction FYGL can also be used for the health care product for daily health care because it is extracted entirely from Ganoderma lucidum fruiting body and does not contain harmful chemical components. The health care products in this invention can contain any of the above-mentioned effective fractions FYGL as their active component and one or more edible carriers. Similarly, the effective fraction FYGL is used either as the sole active component or as a mixture with one or more other effective components in the health care products. Whether the effective fraction FYGL is used alone or in combination, its dosage can be properly adjusted according to the actual situation. Its health care product can be prepared according to conventional methods in this field. Since preparation methods of a health care product containing effective components of traditional Chinese medicine are well-known conventional technologies for those skilled in the art, the methods will not be described in detail herein.

### EXAMPLES

The purpose of the following examples is for the present invention understood well. The invention is not limited to the following examples.

### EXAMPLE 1

10g raw materials of Ganoderma lucidum fruiting body (from Jilin, Leiyunshang Co.) were degreased 3 times with 95 v/v% medicinal ethanol at 70°C for 5 hours each time. The filtered residues from the degreased materials were air-dried, and then the dried products (the degreased products) were treated twice by extraction of 1M sodium carbonate aqueous solution at 20°C for 12 hours. The extraction solution was filtered, and then neutralized to neutral with 1M HCl, and concentrated under vacuum, resulting in the concentrated solution. The solution was dialyzed in water by 1kDa dialysis tube for 2 days, and then the solution in the dialysis tube was concentrated to 1/5 of the original volume, and directly freeze dried, resulting in 0.50g extract (yield 5%).

### EXAMPLE 2

The dialyzed concentrated solution of alkaline extraction was obtained by the method described in Example 1, and was added into 95 v/v% ethanol in volume of five fold of the solution. The solution stood for 12 hours at room temperature, and was centrifuged. The supernatant was collected, and then freeze dried, resulting in the Ganoderma lucidum extract 0.42g (yield 4.2%).

### EXAMPLE 3

10g raw materials of Ganoderma lucidum fruiting body (from Jilin, Leiyunshang Co.) were degreased 3 times with 95 v/v% medicinal ethanol at 78°C for 5 hours each time. The filtered residues from the degreased materials were air-dried, and then the dried products (the degreased products) were treated twice by extraction of 1M sodium hydroxide solution at 20°C for 24 hours. The extraction solution was filtered, and then neutralized to neutral with 1M HCl, and concentrated under vacuum, resulting in the concentrated solution. The solution was dialyzed in water by 1kDa dialysis tube for 2 days, and then concentrated to 1/5 original volume. 95 v/v% ethanol in volume of five fold of the concentrated solution was added into the concentrated solution. The solution stood for 12 hours at room temperature, and was centrifuged. The supernatant was collected, and then freeze dried, resulting in the Ganoderma lucidum extract 0.40g (yield 4.0%).

### EXAMPLE 4

Example 2 was repeated to obtain Ganoderma lucidum extract 0.42g. The extract was dissolved in deionized water, and then absorbed by DE-32 ion exchange resin column with ratio of diameter to height of 25, and eluted gradiently with the eluent of 0.6M NaCl and then 0.2M NaOH. The flow rate was 1 column volume per hour. The elution components, which were eluted by 0.2M NaOH, were neutralized to neutral with HCl, and then probed by phenol-sulfuric acid method. The components with positive reaction of phenol-sulfuric acid method were collected, and then dialyzed by 1kDa dialysis tube to remove the small molecules. Afterward, the solution was vacuum concentrated, and freeze dried, resulting in the eluted components total 0.28g (yield 2.8%).

### EXAMPLE 5

Example 2 was repeated to obtain Ganoderma lucidum extract 0.42g. The extract was dissolved in deionized water, and then absorbed by Sephadex G75 gel chromatography column with ratio of diameter to height of 35, and eluted gradiently with the eluent of 0.2M NaCl. The flow rate was 1 column volume per hour. The elution components were probed by phenol-sulfuric acid method. The components with positive reaction of phenol-sulfuric acid method were collected, and then dialyzed by 1kDa dialysis tube to remove the small molecules. Afterward, the solution was vacuum concentrated, and freeze dried, resulting in the eluted components total 0.06g (yield 0.6%).

### EXAMPLE 6

10g raw materials of Ganoderma lucidum fruiting body (from Jilin, Leiyunshang Co.) were degreased 3 times with 95 v/v% medicinal ethanol (at 78°C) for 4 hours each time. The filtered residues from the degreased materials were air dried, and then the dried products (the degreased products) were refluxed twice in boiling water for 3 hours each time. The extraction solution was filtered, and the filtrate was discarded. The filtered residues of the water extraction were treated twice by extraction of 1M sodium carbonate solution at 20°C for 12 hours. The extraction solution was filtered, and then neutralized to neutral with 1M HCl, and concentrated under vacuum, resulting in the concentrated solution of alkaline extraction. The concentrated solution was dialyzed in water by 1kDa dialysis tube for 2 days, and then the solution in the dialysis tube was concentrated to 1/5 original volume, and 95 v/v% ethanol in volume of five fold of the solution was added. The solution stood for 12 hours at room temperature, and was centrifuged. The supernatant was collected, and then freeze dried, resulting in the Ganoderma lucidum extract 0.31g (yield 3.1%).

### EXAMPLE 7

The dialyzed extracts after alkaline extraction were obtained according to the method described in Example 1. They were filtered by a filter with pore size of 5 micron, and then fractioned by the ultrafiltration membrane with molecular weight cut-off 100kDa, resulting in 1-100kDa components 0.65g (yield 6.5%).

### EXAMPLE 8

The dialyzed extracts after alkaline extraction were obtained according to the method described in Example 3. They were filtered by a filter with pore size of 5 micron, and then fractioned by the ultrafiltration membrane with molecular weight cut-off 100kDa, resulting in 1-100kDa components 0.61g (yield 6.1%).

### EXAMPLE 9

The dialyzed extracts after alkaline extraction were obtained according to the method described in Example 1. They were filtered by a filter with pore size of 5 micron, and then fractioned by the ultrafiltration membrane with molecular weight cut-off 50kDa, resulting in 1-50kDa components 0.30g (yield 3.0%).

### EXAMPLE 10

The dialyzed extracts after alkaline extraction were obtained according to the method described in Example 1. They were filtered by a filter with pore size of 5 micron, and then fractioned by the ultrafiltration membrane with molecular weight cut-off 30kDa, resulting in 1-30kDa components 0.25g (yield 2.5%).

### EXAMPLE 11

1-50kDa components 0.25g were obtained according to the method described in Example 9, and then fractioned by the ultrafiltration membrane with molecular weight cut-off 3kDa to remove the components with molecular weight less than 3kDa, resulting in 3-50kDa components 0.20g (yield 2.0%).

### EXAMPLE 12

1-100kDa components 0.65g were obtained according to the method described in Example 7, and then fractioned by the ultrafiltration membrane with molecular weight cut-off 50kDa to remove the components with molecular weight less than 50kDa, resulting in 50-100kDa components 0.40g (yield 4.0%).

### EXAMPLE 13

1-100kDa components 0.65g were obtained according to the method described in Example 7, and then fractioned by the column chromatography described in Example 4, resulting in the refinement product 0.33g (yield 3.3%).

### EXAMPLE 14

Example 1 was repeated, wherein the difference was that the temperature was 0°C(ice water bath) instead of 20°C during alkaline extraction, and the extraction was performed twice for 24 hours each time, resulting in 0.80g extracts (yield 8.0%).

### EXAMPLE 15

Example 2 was repeated, wherein the difference was that 1M sodium carbonate solution was used during alkaline extraction, and the extraction was performed twice at 10°C for 12 hours each time, resulting in 1.10g extracts (yield 11.0%). The 1.10g extracts were refined according to the method described in Example 5, resulting in the eluted components total 0.08g (yield 0.8%).

### EXAMPLE 16

Example 1 was repeated, wherein the difference was that the temperature was 15°C instead of 20°C during alkaline extraction, and the extraction was performed twice for 12 hours each time. The extract was freeze dried, resulting in 0.62g extracts (yield 6.2%).

### Comparative Example 1

The components with molecular weight greater than 100 kDa obtained in Example 7 were dried, resulting in 0.9g extracts.

### Comparative Example 2

10g raw materials of Ganoderma lucidum fruiting body (from Jilin, Leiyunshang Co.) were degreased 3 times with 95 v/v% medicinal ethanol at 70°C for 5 hours each time. The filtered residues from the degreased materials were air dried, and then the dried products (the degreased products) were refluxed twice in boiling water for 3 hours each time. The extraction solution was filtered, and the filtered residues were discarded. The filtrate was freeze dried, resulting in 0.45g extract.

### Comparative Example 3

Example 16 was repeated, wherein the difference was that the temperature was 25°C instead of 15°C during alkaline extraction, and the alkali solution was changed from 1M sodium carbonate solution to 0.5M NaOH. The extraction was performed twice for 12 hours each time. The solution was dialyzed in water by 1kDa dialysis tube, and then freeze dried, resulting in 0.25g extract (yield 2.5%).

### Comparative Example 4

Example 16 was repeated, wherein the difference was that the temperature was 65°C instead of 15°C during alkaline extraction, and the alkalic solution was changed from 1M sodium carbonate solution to 0.5M NaOH. The extraction was performed twice for 12 hours each time. The solution was dialyzed in water by 3kDa dialysis tube, and then freeze dried, resulting in 0.13g extract (yield 1.3%).

The followings are various analyses for various samples.

### ANALYSIS 1

Infrared spectrum analysis was performed for samples from EXAMPLES and COMPARATIVE EXAMPLES. It was found that the infrared spectra were similar to each other from the examples. The strong absorption band around 3420 cm⁻¹ is a stretching vibration absorption of the hydroxyl groups (O-H), and a band around 2937 cm⁻¹ is attributed to the C-H groups. The absorption bands at 1620 cm⁻¹ and 1400 cm⁻¹ are the typical absorptions of amides I and II in proteins, respectively. The polysaccharide has typical absorption bands in the region of 1200-1000 cm⁻¹ which is dominated by the stretching vibrations of C-OH groups and C-O-C groups. The absorption bands of 1620 cm⁻¹ and 1400 cm⁻¹ in samples from COMPARATIVE EXAMPLE 1 to COMPARATIVE EXAMPLE 4 were significantly weakened.

### ANALYSIS 2

The polysaccharide contents of the extracts from Ganoderma lucidum fruiting body in the mentioned examples and Comparative Examples were analyzed by phenol-sulfuric acid method.

Reagents: concentrated sulfuric acid, an analytical reagent, 95.5wt%. 80wt% phenol: 80g phenol (redistilled analytical reagent) was dissolved in 20g water, storaged light-free in the refrigerator for long-time use. 6wt% phenol: prepared freshly by 80wt% phenol before use. Glucose: an analytical reagent, 98wt%.

Firstly, a work standard curve was plotted for the measurement of sugar contents: 20mg glucose was accurately weighed and dissolved in water in 500mL volumetric flask. 0.4mL, 0.6mL, 0.8mL, 1.0mL, 1.2mL, 1.4mL, 1.6mL and 1.8mL solution was sucked up individually and diluted by water into 2.0mL, and then 1.0mL 6wt% phenol was added, stood for 10 minutes, and then 5mL concentrated sulfuric acid was added, shaked up, stood for 20 minutes at room temperature. The absorption intensity was measured at 490nm, referred to 2.0mL water as a blank. The sugar content standard curve was plotted with sugar in micrograms as x-axis and absorption intensity as y-axis.

Measurement of sugar content of sample: 1.0mL 40µg/mL sample was sucked up, and added with 1mL water and 1.0mL 6 wt% phenol, stood for 10 minutes, and added with 5mL concentrated sulfuric acid, shaked up, and stood for 20 minutes at room temperature. Afterward, the absorbance was measured at 490nm, and the polysaccharide content was evaluated on the standard curve.

The polysaccharide contents of the various samples are listed in Table 2 on the phenol-sulfuric acid method.

**Table 2. Polysaccharide contents of the various samples**

| Sample | Polysaccharide content (wt%) |
|---|---|
| EXAMPLE 1 | 88 ± 2 |
| EXAMPLE 2 | 64 ± 5 |
| EXAMPLE 3 | 60 ± 3 |
| EXAMPLE 4 | 72 ± 2 |
| EXAMPLE 5 | 70 ± 2 |
| EXAMPLE 6 | 61 ± 4 |
| EXAMPLE 7 | 73 ± 3 |
| EXAMPLE 8 | 75 ± 2 |
| EXAMPLE 9 | 75 ± 3 |
| EXAMPLE 10 | 70 ± 3 |
| EXAMPLE 11 | 77 ± 4 |
| EXAMPLE 12 | 73 ± 3 |
| EXAMPLE 13 | 77 ± 3 |
| EXAMPLE 14 | 70 ± 4 |
| EXAMPLE 15 | 62 ± 3 |
| EXAMPLE 16 | 70 ± 2 |
| COMPARATIVE EX. 1 | 95 ± 4 |
| COMPARATIVE EX.2 | 26 ± 1 |
| COMPARATIVE EX.3 | 56 ± 2 |
| COMPARATIVE EX.4 | 28 ± 3 |

### ANALYSIS 3

The protein contents of the extracts from Ganoderma lucidum fruiting body in the mentioned examples and Comparative Examples were analyzed by Lowry assay.
Theory: The peptide bonds in protein chelate Cu²⁺ to form protein-copper complex in alkaline solution. The complex allows the phosphomolybdic acid in phenol reagent reduced, forming a blue compound which has an absorption at 650nm under certain conditions. A standard curve can be plotted on the correlation between absorption intensity at 650nm and protein concentration, therefore used for the evaluation of protein concentration in the sample.

The protein contents of above extracts of Ganoderma lucidum fruiting body are listed in Table 3 on Lowry assay kit.

**Table 3. Protein contents of various samples**

| Sample | Protein content (wt%) |
|---|---|
| EXAMPLE 1 | 6.5 ± 0.7 |
| EXAMPLE 2 | 7.8 ± 0.7 |
| EXAMPLE 3 | 10.5 ± 0.9 |
| EXAMPLE 4 | 14.3 ± 0.5 |
| EXAMPLE 5 | 12.8 ± 1.0 |
| EXAMPLE 6 | 7.9 ± 0.7 |
| EXAMPLE 7 | 8.7 ± 0.4 |
| EXAMPLE 8 | 8.0 ± 0.5 |
| EXAMPLE 9 | 10.9 ± 0.6 |
| EXAMPLE 10 | 11.7 ± 0.7 |
| EXAMPLE 11 | 11.0 ± 0.7 |
| EXAMPLE 12 | 7.9 ± 0.5 |
| EXAMPLE 13 | 16.8 ± 0.9 |
| EXAMPLE 14 | 7.8 ± 0.7 |
| EXAMPLE 15 | 18.8 ± 0.9 |
| EXAMPLE 16 | 7.1 ± 0.8 |
| COMPARATIVE EX. 1 | 2.5 ± 0.4 |
| COMPARATIVE EX.2 | 2.2 ± 0.4 |
| COMPARATIVE EX.3 | 5.3 ± 0.4 |
| COMPARATIVE EX.4 | 3.5 ± 0.4 |

### ANALYSIS 4

The inhibitory concentration on protein tyrosine phosphatase-1B (PTP1B) activity was measured for the above extracts of Ganoderma lucidum fruiting body from EXAMPLES and COMPARATIVE EXAMPLES.

Disodium *p*-nitrophenyl phosphate (*p*NPP) is a soluble substrate of alkaline phosphatase. It is dephosphated by the catalysis of human protein tyrosine phosphatase-1B (PTP1B, Sigma) to produce a yellow soluble *p*-nitrophenol which absorbs light at 405nm. The reduced absorptions at 405nm are measured by microplate reader after FYGL of a series of concentrations are added into PTP1B and *p*NPP solution for evaluation of the inhititory efficiency of FYGL on PTP1B activity, referred to the positive control of sodium vanadate. The buffer is 50mM Tris and 150mM sodium chloride at pH 8.0.

The half inhibitory concentrations of IC₅₀ on PTP1B activity are listed in Table 4 for various samples at PTP1B concentration of 25 µg/mL.

**Table 4. Half inhibitory concentraion of IC₅₀ on PTP1B activity for various samples**

| sample | half inhibitory concentration of IC₅₀ (µg/mL) |
|---|---|
| EXAMPLE 1 | 60 |
| EXAMPLE 2 | 51 |
| EXAMPLE 3 | 26 |
| EXAMPLE 4 | 18 |
| EXAMPLE 5 | 16 |
| EXAMPLE 6 | 45 |
| EXAMPLE 7 | 33 |
| EXAMPLE 8 | 35 |
| EXAMPLE 9 | 26 |
| EXAMPLE 10 | 24 |
| EXAMPLE 11 | 25 |
| EXAMPLE 12 | 43 |
| EXAMPLE 13 | 11 |
| EXAMPLE 14 | 42 |
| EXAMPLE 15 | 1.2 |
| EXAMPLE 16 | 50 |
| COMPARATIVE Ex.1 | 229 |
| COMPARATIVE Ex.2 | > 300 |
| COMPARATIVE Ex.3 | 101 |
| COMPARATIVE Ex.4 | > 300 |

### ANALYSIS 5

The inhibitory efficiency on α-amylase activity was measured for the extract of Ganoderma lucidum fruiting body from Example 13.

3,5-dinitrosalicylic acid method (Bernfeld method) is commonly used for screening the amylase inhibitors in vitro. The soluble starch is hydrolyzed by α-amylase, producing the reduction group. 3,5-dinitrosalicylic acid is reduced into the nitro amino salicylic acid which is orange color in excess NaOH solution and absorb light at 540nm. The α-amylase inhibitor can inhibit the starch hydrolysis, leading to the decrease in A₅₄₀ₙₘ intensity, therefore, the inhibitory concentration of the studied sample on the amylase can be estimated, referred to acarbose as positive control.

The half inhibitory concentration IC₅₀ of 15U/mL sample from Example 13 is 83 ± 10 µg/mL on the α-amylase activity.

### ANALYSIS 6

The inhibitory efficiency on α-glucosidase activity was measured for the extract of Ganoderma lucidum fruiting body from Example 13.

p-nitrophenyl-α-D-glucopyranoside (pNPG) is a soluble substrate. It is dephosphated by the catalysis of α-glucosidase to produce a yellow soluble p-nitrophenol which absorbs light at 405nm. The reduced absorption at 405nm is measured by microplate reader for evaluation of the inhititory efficiency of FYGL on the α-glucosidase activity, referred to the positive control of acarbose.

The half inhibitory concentration IC₅₀ of 0.03U/mL sample from Example 13 is 97 ± 10 µg/mL on the α-glucosidase activity.

### ANALYSIS 7

The extract from Example 13 was trialed for its drug potency on STZ-induced type 2 diabetic model rats in Center for Drug Safety Evaluation, Shanghai University of Traditional Chinese Medicine. The results are listed in Table 5.

**Table 5. Drug potency of the sample from Example 13 on STZ-induced type 2 diabetic model rats**

| group | fasting blood glucose (mM) | | |
|---|---|---|---|
| | 10 days | 20 days | 30 days |
| normal | 4.8 ± 0.3 | 4.0 ± 0.9 | 6.8 ± 0.7 |
| type 2 diabetic control | 23 ± 3 | 25 ± 4 | 22 ± 5 |
| Low dose of FYGL | 19 ± 4 | 21 ± 4 | 20 ± 4 |
| high dose of FYGL | 18 ± 4(#) | 17 ± 3(#) | 15 ± 1 (##) |
| Metformin | 14 ± 4(#) | 18 ± 5(#) | 13 ± 2 (##) |
| rosiglitazone | 14 ± 5(#) | 14 ± 4(#) | 12 ± 3 (##) |

FYGL was orally administered for various groups as: type 2 diabetes control group with saline 20mL/kg; type 2 diabetes group with low dose of FYGL 50mg/kg; type 2 diabetes group with high dose of FYGL 150mg/kg; metformin group with metformin 200mg/kg; rosiglitazone group with rosiglitazone 3mg/kg. Numbers of rats in each group are 10.

After several weeks, the fasting blood glucose levels in type 2 diabetes groups administering FYGL were decreased significantly than that in control group, and comparable with that in the positive groups administering metformin and rosiglitazone. The symbol # indicates significant difference of the fasting blood glucose level with p < 0.05 and ## indicates very significant difference with p < 0.01, referred to that of type 2 diabetes control group.

### ANALYSIS 8

¹H NMR experiments were performed for those samples from EXAMPLES and COMPARATIVE EXAMPLES as the following conditions:
INSTRUMENT: Bruker DMX500 NMR spectrometer (Germany) with ¹H resonance frequency of 500MHz, water peak suppression with WATERGATE gradient field pulse sequence, ¹H 90° pulse width of 9.4µs, acquisition time of 2.044s, acquisition points of 32K, and number of scan of 128.
SAMPLE PREPARATION: 20mg sample dissolved in 0.5mL deuterium water in 5mm diameter NMR tube.
CONDITIONS: room temperature, ¹H chemical shifts referred to tetramethylsilane as an external standard set at 0.000 ppm.

¹H NMR spectra of all samples include the peaks around chemical shifts δ 0.9, 1.2, 1.4, 1.6, 2.0, 2.3, 2.7, 3.4, 3.5, 3.6, 3.8, 3.9, 4.0, 4.2, 4.3, 4.5, 4.7, 4.8, 5.0, 5.3, 5.9, 6.0 and 6.6-7.6 ppm. Figure 1 is ¹H NMR spectrum of sample from EXAMPLE 1. Comparisons of the ratio (ratio I) of peak integral area at chemical shifts δ=3.6-3.4ppm to that at δ=3.4-3.2ppm with the ratio (ratio II) of peak integral area at chemical shifts δ=3.0∼1.0ppm to that at δ=3.4∼3.2ppm for samples from EXAMPLES and COMPARATIVE EXAMPLES are summarized in Table 6.

**Table 6. Comparisons of ¹H NMR peak integral area ratios for samples from EXAMPLES and Comparative Examples**

| Sample | ratio I | ratio II |
|---|---|---|
| EXAMPLE 1 | 0.75 | 1.45 |
| EXAMPLE 2 | 0.82 | 1.12 |
| EXAMPLE 3 | 1.14 | 2.73 |
| EXAMPLE 4 | 1.27 | 3.32 |
| EXAMPLE 5 | 1.29 | 3.50 |
| EXAMPLE 6 | 0.90 | 1.50 |
| EXAMPLE 7 | 1.08 | 2.05 |
| EXAMPLE 8 | 1.04 | 2.07 |
| EXAMPLE 9 | 1.10 | 2.89 |
| EXAMPLE 10 | 1.18 | 3.05 |
| EXAMPLE 11 | 1.16 | 2.93 |
| EXAMPLE 12 | 0.93 | 1.64 |
| EXAMPLE 13 | 1.30 | 3.39 |
| EXAMPLE 14 | 0.95 | 1.64 |
| EXAMPLE 15 | 1.45 | 3.79 |
| EXAMPLE 16 | 0.78 | 1.23 |
| COMPARATIVE EX. 1 | 0.38 | 0.55 |
| COMPARATIVE EX.2 | 0.27 | 0.50 |
| COMPARATIVE EX.3 | 0.59 | 0.74 |
| COMPARATIVE EX.4 | 0.30 | 0.47 |

### ANALYSIS 9

The monosaccharide components were analyzed for the extract from Ganoderma lucidum fruiting body from EXAMPLE 13.

The monosaccharide components were analyzed by ion chromatography. The hydrolysis of polysaccharide and the chromatography conditions are described as below:

### (1) Hydrolysis of polysaccharide

1.0mg polysaccharide sample with 1.0mL 2M trifluoroacetic acid was sealed in a test tube at 100°C for 2-4 hours for hydrolysis reaction; and then opened the tube, the contents in tube were dried with nitrogen, and dissolved in water to volume of 2.0mL, and then filtered by 0.2 µm filter for analysis.

### (2) Chromatography conditions

25µL sample was injected and the monosaccharide components were separated by a CarboPac^{™} PA10 anion-exchange chromatography column (length × diameter of 250 mm × 2 mm),sugar guard column (length × diameter of 50 mm × 2 mm) with AminoPAC Trap column (length × diameter of 50 mm × 2 mm) using 14.0mM NaOH as eluent at flow rate of 0.20 mL/min at 30°C. Before measurement, the chromatography column was regenerated by 0.2mM NaOH for 10.0 hours, and then balanced by 14.0mM NaOH for 2 hours.

The monosaccharide components and their contents for sample from EXAMPLE 13 are summarized in Table 7.

**Table 7. The monosaccharide components and their contents for sample from EXAMPLE 13**

| Monosaccharide | Content (%) |
|---|---|
| glucose | 78.12 |
| arabinose | 9.72 |
| xylose | 7.50 |
| rhamnose | 2.26 |
| galactose | 1.96 |
| fructose | 0.44 |

### ANALYSIS 10

The amino acid residues were analyzed for the extract from Ganoderma lucidum fruiting body from EXAMPLE 13.

The amino acid components were analyzed using amino acid analyzer with traditional cation exchange chromatography and ninhydrin derivation after column for the hydrolyzed protein and free amino acids.

2mg sample and 2mL HCl were together in a test tube and purged with nitrogen gas for more than 5min, and then the test tube was sealed and placed in the drying oven at 110°C for 22-24 hours for hydrolysis. Afterward, the sample was cooled down to room temperature and diluted up to volume of 10mL, and then filtered. 5mL filtrate was dried at 55°C in vacuum desiccator, and then dissolved in 1.0mL ultra-pure water, shaked up. pH was adjusted to 1.7-2.2 (measured with precision pH paper) by 1M HCl or NaOH, and then filtered and diluted for the analysis.
AMINO ACID ANALYZER: Hitachi automatic amino acid analyzer L-8900

The amino acid components and their contents for sample from EXAMPLE 13 are summarized in Table 8.

**Table 8. The amino acid components and their contents for sample from EXAMPLE 13**

| Amino acid | Abbreviation | Content (%) |
|---|---|---|
| aspartic acid, asparagine | Asp, Asn | 13.03 |
| glycine | Gly | 10.96 |
| glutamic acid, glutamine | Glu, Gln | 10.15 |
| alanine | Ala | 8.72 |
| serine | Ser | 8.65 |
| threonine | Thr | 7.51 |
| leucine | Leu | 5.98 |
| phenylalanine | Phe | 5.15 |
| valine | Val | 4.78 |
| proline | Hydro Pro | 4.11 |
| isoleucine | Ile | 3.84 |
| ammonia | NH3 | 3.44 |
| tyrosine | Tyr | 3.05 |
| arginine | Arg | 2.64 |
| lysine | Lys | 2.63 |
| histidine | His | 2.10 |
| ornithine hydrochloride | Ornithine hydrochloride | 1.73 |
| cysteine | Cys | 0.71 |
| methionine | Met | 0.40 |
| γ-aminobutyric acid | GABA | 0.40 |

The invention has been illustrated through the above Examples. But it should be understood that those Examples are only used for the purposes of illustration and are not intended to limit the invention to the above described Examples. The protection scope of the present invention is defined by the claims and their equivalent range.

## Claims

1. An effective fraction FYGL in Ganoderma lucidum fruiting body, wherein the effective fraction FYGL is an extract from Ganoderma lucidum fruiting body, and has a half inhibitory concentration of IC₅₀ equal to or lower than 80 µg/mL on protein tyrosine phosphatase-1B activity, wherein ¹H NMR spectrum of the effective fraction FYGL comprises peaks around the chemical shifts δ 0.9, 1.2, 1.4, 1.6, 2.0, 2.3, 2.7, 3.4, 3.5, 3.6, 3.8, 3.9, 4.0, 4.2, 4.3, 4.5, 4.7, 4.8, 5.0, 5.3, 5.9, 6.0 and 6.6-7.6 ppm, as measured by using deuterium water as the solvent with the chemical shift of tetramethylsilane as an external or internal standard set at 0.0 ppm and wherein in the 1H NMR spectrum, the ratio of peak integral area at chemical shift δ=3.6-3.4ppm to peak integral area at δ=3.4-3.2ppm is 0.5-1.5; and the ratio of peak integral area at chemical shift δ=3.0-1.0ppm to peak integral area at δ=3.4-3.2ppm is 0.5-4.0,
wherein the effective fraction FYGL has a content of proteins of 3 wt.%-20 wt.%, as measured by Lowry assay,
wherein the effective fraction FYGL is prepared by the following method comprising: a degreased product of Ganoderma lucidum fruiting body is extracted by alkaline extraction at 0-20°C, preferably at 4-15°C, or the degreased product is extracted by water to obtain filtered residues and/or residues which are then extracted by alkaline extraction at 0-20°C, preferably at 4-15°C, so as to provide an extract from alkaline extraction of Ganoderma lucidum fruiting body; the extract is dialyzed to remove small molecules; and then the dialyzed extract is directly subject to a drying treatment so as to obtain the effective fraction FYGL, or the dialyzed extract is subject to a separating treatment before the drying treatment, so as to obtain the effective fraction FYGL.

2. The effective fraction FYGL according to claim 1, wherein the effective fraction FYGL further contains polysaccharides comprising monosaccharide units of glucose, arabinose, xylose, rhamnose, galactose and fructose.

3. The effective fraction FYGL according to claim 1, wherein the degreased product is obtained by degreasing the Ganoderma lucidum fruiting body using ethanol or the aqueous solution ethanol.

4. The effective fraction FYGL according to any of claims 1-3, wherein the alkaline extraction is carried out by using an aqueous solution containing at least one selected from sodium carbonate, sodium bicarbonate, potassium hydroxide, sodium hydroxide or ammonia, preferably by using 0.5M or higher of the aqueous solution of sodium carbonate or sodium bicarbonate, or 0.1-2M aqueous solution of potassium hydroxide, sodium hydroxide or ammonia.

5. The effective fraction FYGL according to any of claims 1-4, wherein the dialysis is conducted using dialysis tube of 1kDa-3kDa to remove the small molecules with molecular weight less than 1kDa-3kDa

6. The effective fraction FYGL according to any of claims 1-5, wherein the separating treatment comprises:
(1) carrying out an alcohol precipitation of the dialyzed extract so as to obtain a supernatant; or
(2) fractionating the dialyzed extract in the manner of ultrafiltration by using ultrafiltration membrane with its molecular weight cut-off of 100 kDa or less, so as to remove those with molecular weight higher than the cut-off value.

7. The effective fraction FYGL according to claim 6, wherein the alcohol precipitation is performed by precipitating the dialyzed extract using an aqueous solution of ethanol, and then collecting the supernatant.

8. The effective fraction FYGL according to claim 6 or 7, wherein the effective fraction FYGL is substantially composed of materials with molecular weights from 1kDa to 100kDa.

9. The effective fraction FYGL according to any of claims 6-8, wherein before the drying treatment and after the separating treatment, the method further includes performing a column chromatography with the extract.

10. The effective fraction FYGL according to claim 9, wherein the column chromatography is performed by using at least one selected from DEAE-cellulose, DEAE-Sephadex, DEAE-Sepharose or macroporous adsorption resin, preferably with eluents of 0.1-2M NaCl aqueous solution and then 0.1-2M NaOH aqueous solution.

11. Use of the effective fraction FYGL in Ganoderma lucidum fruiting body according to any of claims 1 to 10 as the sole active component for manufacturing a pharmaceutical, which is used for the treatment or prevention of at least one selected from diabetes and metabolic syndrome diseases; or for the treatment or prevention of at least one disease associated with diabetes or metabolic syndrome, selected from atheriosclerosis, atherosclerosis, obesity, hypertension, hyperlipidemia, fatty liver disease, kidney disease, neurological disease, retinopathy, foot ulcer or cataract; or for the treatment of at least one disease selected from hyperlipidemia, obesity or cachectic disease.

12. A pharmaceutical preparation, comprising the effective fraction FYGL in Ganoderma lucidum fruiting body according to any of claims 1 to 10 as an active component and one or more pharmaceutically acceptable carriers, and excluding any other extract from Ganoderma lucidum fruiting body.

13. A health care product comprising the effective fraction FYGL in Ganoderma lucidum fruiting body according to any of claims 1 to 10 as an active component and one or more edible carriers, and excluding any other extract from Ganoderma lucidum fruiting body.

14. A method for the extraction of effective fraction FYGL from Ganoderma lucidum fruiting body as claimed in claim 1, wherein the method comprises: a degreased product of Ganoderma lucidum fruiting body is extracted by alkaline extraction at 0-20°C, preferably at 4-15°C, or the degreased product is extracted by water to obtain filtered residues and/or residues which are then extracted by alkaline extraction at 0-20°C, preferably at 4-15°C, so as to provide an extract from alkaline extraction of Ganoderma lucidum fruiting body; the extract is dialyzed to remove small molecules; and then the dialyzed extract is directly subject to a drying treatment so as to obtain the effective fraction FYGL, or the dialyzed extract is subject to a separating treatment before the drying treatment, so as to obtain the effective fraction FYGL.

## Patentansprüche

1. Wirksame Fraktion FYGL im Fruchtkörper von Ganoderma-lucidum, wobei die wirksame Fraktion FYGL ein Extrakt des Fruchtkörpers von Ganoderma-lucidum ist und eine mittlere inhibitorische Konzentration IC₅₀ gleich oder unter 80 µg/mL für Proteintyrosinphosphatase-1B-Aktivität aufweist, wobei das ¹H-NMR-Spektrum der wirksamen Fraktion FYGL Spitzen um die chemischen Verschiebungen δ 0,9, 1,2, 1,4, 1,6, 2,0, 2,3, 2,7, 3,4, 3,5, 3,6, 3,8, 3,9, 4,0, 4,2, 4,3, 4,5, 4,7, 4,8, 5,0, 5,3, 5,9, 6,0 und 6,6-7,6 ppm aufweist, gemessen unter Verwendung von Deuteriumwasser als das Lösungsmittel, mit der chemischen Verschiebung von Tetramethylsilan als ein externer oder interner Standard, der auf 0,0 ppm festgelegt ist, und wobei in dem ¹H-NMR-Spektrum das Verhältnis der Spitzenintegrationsfläche bei der chemischen Verschiebung δ=3,6-3,4 ppm zur Spitzenintegrationsfläche bei δ=3,4-3,2 ppm 0,5-1,5 beträgt; und das Verhältnis der Spitzenintegrationsfläche bei der chemischen Verschiebung δ=3,0-1,0 ppm zur Spitzenintegrationsfläche bei δ=3,4-3,2 ppm 0,5-4,0 beträgt,
wobei die wirksame Fraktion FYGL einen Proteingehalt von 3 Gew.%-20 Gew.% aufweist, gemessen mittels Lowry-Assay,
wobei die wirksame Fraktion FYGL durch das folgende Verfahren hergestellt wird, umfassend: Extrahieren eines entfetteten Produkts von Ganoderma-lucidum durch Alkaliextraktion bei 0-20 °C, vorzugsweise bei 4-15 °C, oder Extrahieren des entfetteten Produkts durch Wasser, um gefilterte Rückstände und/oder Rückstände zu erlangen, die dann durch Alkaliextraktion bei 0-20 °C, vorzugsweise bei 4-15 °C extrahiert werden, um ein Extrakt durch Alkaliextraktion aus dem Fruchtkörper von Ganoderma-lucidum bereitzustellen; Dialysieren des Extrakts zum Entfernen kleiner Moleküle; und dann direktes Unterziehen des dialysierten Extrakts einer Trocknungsbehandlung, um die wirksame Fraktion FYGL zu erlangen, oder Unterziehen des dialysierten Extrakts einer Trennbehandlung vor der Trocknungsbehandlung, um die wirksame Fraktion FYGL zu erlangen.

2. Wirksame Fraktion FYGL nach Anspruch 1, wobei die wirksame Fraktion FYGL ferner Polysaccharide enthält, die Monosaccharideinheiten von Glucose, Arabinose, Xylose, Rhamnose, Galactose und Fructose umfassen.

3. Wirksame Fraktion FYGL nach Anspruch 1, wobei das entfettete Produkt durch Entfetten des Fruchtkörpers von Ganoderma-lucidum mit Ethanol oder wässriger Ethanollösung erlangt wird.

4. Wirksame Fraktion FYGL nach einem der Ansprüche 1-3, wobei die Alkaliextraktion unter Verwendung einer wässrigen Lösung durchgeführt wird, die wenigstens eins, ausgewählt aus Natriumcarbonat, Natriumbicarbonat, Kaliumhydroxid, Natriumhydroxid oder Ammoniak, enthält, vorzugsweise unter Verwendung von 0,5 M oder mehr der wässrigen Lösung von Natriumcarbonat oder Natriumbicarbonat, oder 0,1-2 M wässriger Lösung von Kaliumhydroxid, Natriumhydroxid oder Ammoniak.

5. Wirksame Fraktion FYGL nach einem der Ansprüche 1-4, wobei die Dialyse unter Verwendung einer Dialyseröhre von 1 kDa-3 kDa durchgeführt wird, um kleine Moleküle mit einem Molekulargewicht von weniger als 1 kDa-3 kDa zu entfernen.

6. Wirksame Fraktion FYGL nach einem der Ansprüche 1-5, wobei die Trennbehandlung Folgendes umfasst:
(1) Durchführen einer Alkoholausfällung des dialysierten Extrakts, um einen Überstand zu erlangen; oder
(2) Fraktionieren des dialysierten Extrakts nach Art der Ultrafiltration durch Verwendung einer Ultrafiltrationsmembran, deren Molekulargewichthöchstgrenze 100 kDa oder weniger beträgt, um diejenigen mit einem höheren Molekulargewicht als die Höchstgrenze zu entfernen.

7. Wirksame Fraktion FYGL nach Anspruch 6, wobei die Alkoholausfällung durch Ausfällen des dialysierten Extrakts unter Verwendung einer wässrigen Lösung von Ethanol und anschließendes Auffangen des Überstands durchgeführt wird.

8. Wirksame Fraktion FYGL nach Anspruch 6 oder 7, wobei die wirksame Fraktion FYGL im Wesentlichen aus Materialien mit Molekulargewicht von 1 kDa bis 100 kDa besteht.

9. Wirksame Fraktion FYGL nach einem der Ansprüche 6-8, wobei das Verfahren vor der Trocknungsbehandlung und nach der Trennbehandlung das Durchführen einer Säulenchromatographie mit dem Extrakt beinhaltet.

10. Wirksame Fraktion FYGL nach Anspruch 9, wobei die Säulenchromatographie unter Verwendung von wenigstens einem, ausgewählt aus DEAE-Cellulose, DEAE-Sephadex, DEAE-Sepharose oder makroporösem Adsorptionsharz durchgeführt wird, vorzugsweise mit den Eluenten 0,1-2 M wässrige NaCl-Lösung und dann 0,1-2 M wässrige NaOH-Lösung.

11. Verwendung der wirksamen Fraktion FYGL im Fruchtkörper von Ganoderma-lucidum nach einem der Ansprüche 1 bis 10 als einzige aktive Komponente zum Herstellen eines Pharmazeutikums, das zur Behandlung oder Prävention von wenigstens einem, ausgewählt aus den Krankheiten Diabetes und metabolisches Syndrom; oder zur Behandlung oder Prävention von wenigstens einer Krankheit im Zusammenhang mit Diabetes oder metabolischem Syndrom, ausgewählt aus Arteriosklerose, Atherosklerose, Fettleibigkeit, Bluthochdruck, Hyperlipidämie, Fettleber, Nierenkrankheit, neurologischer Krankheit, Retinopathie, Fußgeschwür oder Katarakt; oder zur Behandlung oder Prävention von wenigstens einer Krankheit ausgewählt aus Hyperlipidämiie, Fettleibigkeit oder Kachexie verwendet wird.

12. Pharmazeutische Zubereitung, umfassend die wirksame Fraktion FYGL im Fruchtkörper von Ganoderma-lucidum nach einem der Ansprüche 1 bis 10 als eine aktive Komponente und einen oder mehrere pharmazeutisch akzeptable Träger, und ohne jeglichen anderen Extrakt aus dem Fruchtkörper von Ganoderma-lucidum.

13. Gesundheitsprodukt, umfassend die wirksame Fraktion FYGL im Fruchtkörper von Ganoderma-lucidum nach einem der Ansprüche 1 bis 10 als einen Wirkstoff und einen oder mehrere essbare Träger, und ohne jeglichen anderen Extrakt aus dem Fruchtkörper von Ganoderma-lucidum.

14. Verfahren zur Extraktion von wirksamer Fraktion FYGL aus dem Fruchtkörper von Ganoderma-lucidum nach Anspruch 1, wobei das Verfahren Folgendes umfasst: Extrahieren eines entfetteten Produkts von Ganoderma-lucidum durch Alkaliextraktion bei 0-20 °C, vorzugsweise bei 4-15 °C, oder Extrahieren des entfetteten Produkts durch Wasser, um gefilterte Rückstände und/oder Rückstände zu erlangen, die dann durch Alkaliextraktion bei 0-20 °C, vorzugsweise bei 4-15 °C extrahiert werden, um ein Extrakt durch Alkaliextraktion aus dem Fruchtkörper von Ganoderma-lucidum bereitzustellen; Dialysieren des Extrakts zum Entfernen kleiner Moleküle; und dann direktes Unterziehen des dialysierten Extrakts einer Trocknungsbehandlung, um die wirksame Fraktion FYGL zu erlangen, oder Unterziehen des dialysierten Extrakts einer Trennbehandlung vor der Trocknungsbehandlung, um die wirksame Fraktion FYGL zu erlangen.

## Revendications

1. Fraction active FYGL du corps fructifère de *Ganoderma lucidum,* laquelle fraction active FYGL est un extrait du corps fructifère de *Ganoderma lucidum* et présente une concentration inhibitrice médiane CI₅₀ de l'activité de la protéine à tyrosine phosphatase-1B inférieure ou égale à 80 µg/mL, laquelle fraction active FYGL présente un spectre RMN ¹H comprenant des pics autour des déplacements chimiques δ 0,9, 1,2, 1,4, 1,6, 2,0, 2,3, 2,7, 3,4, 3,5, 3,6, 3,8, 3,9, 4,0, 4,2, 4,3, 4,5, 4,7, 4,8, 5,0, 5,3, 5,9, 6,0 et 6,6-7,6 ppm, mesurés dans l'eau deutérée en tant que solvant, par rapport au tétraméthylsilane, utilisé comme référence externe ou interne et dont le déplacement chimique a été réglé à 0,0 ppm, lequel spectre RMN ¹H montre un ratio de l'aire intégrale du pic à δ = 3,6-3,4 ppm à l'aire intégrale du pic à δ = 3,4-3,2 ppm compris entre 0,5 et 1,5 ; et un ratio de l'aire intégrale du pic à δ = 3,0-1,0 ppm à l'aire intégrale du pic à δ = 3,4-3,2 ppm compris entre 0,5 et 4,0,
laquelle fraction active FYGL présente un taux de protéines compris entre 3 et 20 % m/m, mesuré par la méthode de Lowry,
laquelle fraction active FYGL est préparée selon une méthode comprenant les étapes suivantes : extraire un produit délipidé du corps fructifère de *Ganoderma lucidum* par extraction alcaline à 0-20 °C, de préférence à 4-15 °C, ou extraire le produit délipidé à l'eau afin d'obtenir des résidus filtrés et/ou des résidus que l'on soumet ensuite à une extraction alcaline à 0-20 °C, de préférence à 4-15 °C, de manière à obtenir un extrait issu de l'extraction alcaline du corps fructifère de *Ganoderma lucidum* ; dialyser l'extrait afin d'éliminer les molécules de petite taille ; puis soumettre l'extrait dialysé à un traitement de séchage afin d'obtenir la fraction active FYGL, ou soumettre l'extrait dialysé à un traitement de séparation préalable au séchage afin d'obtenir la fraction active FYGL.

2. Fraction active FYGL conforme à la revendication 1, laquelle fraction active FYGL contient en outre des polysaccharides comprenant des unités monosaccharidiques de glucose, d'arabinose, de xylose, de rhamnose, de galactose et de fructose.

3. Fraction active FYGL conforme à la revendication 1, le produit délipidé étant obtenu par délipidation du corps fructifère de *Ganoderma lucidum* à l'aide d'éthanol ou d'une solution aqueuse d'éthanol.

4. Fraction active FYGL conforme à l'une quelconque des revendications 1 à 3, l'extraction alcaline étant réalisée à l'aide d'une solution aqueuse contenant au moins un composé choisi parmi le carbonate de sodium, le bicarbonate de sodium, l'hydroxyde de potassium, l'hydroxyde de sodium ou l'ammoniac, de préférence à l'aide d'une solution aqueuse de concentration au moins égale à 0,5 M de carbonate de sodium ou de bicarbonate de sodium, ou d'une solution aqueuse de concentration comprise entre 0,1 et 2 M d'hydroxyde de potassium, d'hydroxyde de sodium ou d'ammoniaque.

5. Fraction active FYGL conforme à l'une quelconque des revendications 1 à 4, la dialyse étant réalisée à l'aide d'un tube de dialyse de 1 à 3 kDa de manière à éliminer les molécules dont la masse moléculaire est inférieure à 1 à 3 kDa.

6. Fraction active FYGL conforme à l'une quelconque des revendications 1 à 5, le traitement de séparation comprenant les étapes consistant à :
(1) procéder à une précipitation alcoolique de l'extrait dialysé de manière à obtenir un surnageant; ou
(2) fractionner l'extrait dialysé par ultrafiltration à l'aide d'une membrane d'ultrafiltration dont le seuil de coupure correspond à une masse moléculaire d'au plus 100 kDa, de manière à éliminer les molécules dont la masse moléculaire est inférieure au seuil de coupure.

7. Fraction active FYGL conforme à la revendication 6, la précipitation alcoolique étant réalisée par précipitation de l'extrait dialysé à l'aide d'une solution aqueuse d'éthanol suivie d'une récupération du surnageant.

8. Fraction active FYGL conforme à la revendication 6 ou 7, laquelle fraction active FYGL est en grande partie composée de substances dont la masse moléculaire est comprise entre 1 kDa et 100 kDa.

9. Fraction active FYGL conforme à l'une quelconque des revendications 6 à 8, la méthode comprenant en outre, après le traitement de séparation et avant le traitement de séchage, une chromatographie sur colonne de l'extrait.

10. Fraction active FYGL conforme à la revendication 9, la chromatographie sur colonne étant réalisée à l'aide d'au moins une résine choisie parmi la DEAE-cellulose, la DEAE-Sephadex, la DEAE-Sepharose ou une résine d'adsorption macroporeuse, les éluents étant de préférence une solution aqueuse de 0,1-2 M de NaCl puis une solution aqueuse de 0,1-2 M de NaOH.

11. Utilisation de la fraction active FYGL du corps fructifère de *Ganoderma lucidum* conforme à l'une quelconque des revendications 1 à 10 en tant qu'unique composant actif dans la fabrication d'un produit pharmaceutique, lequel sert au traitement ou à la prévention d'au moins une maladie sélectionnée dans le groupe constitué par le diabète et le syndrome métabolique ; ou bien au traitement ou à la prévention d'au moins une maladie associée au diabète et au syndrome métabolique et sélectionnée dans le groupe constitué par l'artériosclérose, l'athérosclérose, l'obésité, l'hypertension, l'hyperlipidémie, la stéatose hépatique, une maladie rénale, une maladie neurologique, une retinopathie, un ulcère du pied ou la cataracte ; ou encore au traitement d'au moins une maladie sélectionnée dans le groupe constitué par l'hyperlipidémie, l'obésité, ou la cachexie.

12. Préparation pharmaceutique comprenant la fraction active FYGL du corps fructifère de *Ganoderma lucidum* conforme à l'une quelconque des revendications 1 à 10 en tant que composant actif ainsi qu'un ou plusieurs véhicules pharmacologiquement admissibles, et ne comprenant aucun autre extrait du corps fructifère de *Ganoderma lucidum.*

13. Produit de soin de santé comprenant la fraction active FYGL du corps fructifère de *Ganoderma lucidum* conforme à l'une quelconque des revendications 1 à 10 en tant que composant actif ainsi qu'un ou plusieurs véhicules pharmacologiquement admissibles, mais ne comprenant aucun autre extrait du corps fructifère de *Ganoderma lucidum.*

14. Méthode d'extraction de la fraction active FYGL du corps fructifère de *Ganoderma lucidum* conforme à la revendication 1, laquelle méthode comprend les étapes suivantes : extraire un produit délipidé du corps fructifère de *Ganoderma lucidum* par extraction alcaline à 0-20 °C, de préférence à 4-15 °C, ou extraire le produit délipidé à l'eau afin d'obtenir des résidus filtrés et/ou des résidus que l'on soumet ensuite à une extraction alcaline à 0-20 °C, de préférence à 4-15 °C, de manière à obtenir un extrait issu de l'extraction alcaline du corps fructifère de *Ganoderma lucidum* ; dialyser l'extrait afin d'éliminer les molécules de petite taille ; puis soumettre l'extrait dialysé à un traitement de séchage afin d'obtenir la fraction active FYGL, ou soumettre l'extrait dialysé à un traitement de séparation préalable au séchage afin d'obtenir la fraction active FYGL .
